# EUROPEAN PATENT SPECIFICATION

(11) **EP 4 146 111 B1**
(45) Date of publication and mention of the grant of the patent: **08.07.2026**
(21) Application number: 21720943.6
(22) Date of filing: 08.04.2021
(51) Int. Cl.: A61B 34/10

(54) **SURGICAL SYSTEM FOR REVISION ORTHOPEDIC SURGICAL PROCEDURES**
CHIRURGISCHES SYSTEM ZUR REVISION ORTHOPÄDISCHER CHIRURGISCHER EINGRIFFE
SYSTÈME CHIRURGICAL POUR INTERVENTIONS CHIRURGICALES ORTHOPÉDIQUES DE RÉVISION

(30) Priority: 04.05.2020 US 202063019604 P
(43) Date of publication of application: 15.03.2023
(73) Proprietor: Howmedica Osteonics Corp., Mahwah, NJ 07430 (US)
(72) Inventor: SIMOES, Vincent Abel Maurice, 29280 Plouzané (FR); MAILLE, Florence Delphine Muriel, 29280 Plouzané (FR)
(74) Representative: Zimmermann & Partner Patentanwälte mbB
(86) International application number: PCT/US2021/026441
(87) International publication number: WO 2021/225738

(56) References cited:
- US-A- 5 480 439
- US-A1- 2006 224 088
- US-A1- 2009 221 922
- US-A1- 2018 014 891
- US-A1- 2019 380 792

## Description

This application claims the benefit of US Provisional Patent Application No. 63/019,604, filed 4 May 2020.

### BACKGROUND

Surgical joint repair procedures involve repair and/or replacement of a damaged or diseased joint. Many times, a surgical joint repair procedure, such as joint arthroplasty as an example, involves replacing the damaged joint with a prosthetic that is implanted into the patient's bone. Proper selection of a prosthetic that is appropriately sized and shaped and proper positioning of that prosthetic to ensure an optimal surgical outcome can be challenging. To assist with positioning, the surgical procedure often involves the use of surgical instruments to control the shaping of the surface of the damaged bone and cutting or drilling of bone to accept the prosthetic.

Today, visualization tools are available to surgeons that use three-dimensional modeling of bone characteristics (e.g., bone shapes) to facilitate preoperative planning for joint repairs and replacements. These tools can assist surgeons with the design and/or selection of surgical guides and implants that closely match the patient's anatomy and can improve surgical outcomes by customizing a surgical plan for each patient.

Document US2018014891 A1 discloses a method for performing a revision surgery using a robotic-assisted surgery system that includes determining information related to an interface area between an implant component and a bone, and generating a planned virtual boundary associated with a portion of the interface area to be removed in a representation of the implant and the bone, based at least in part on the information related to the interface area.

Document US5480439 A discloses a method of evaluating bone density around a radiolucent composite prosthesis that establishes an implant boundary by fitting a stored template to radio-opaque reference markers embedded in the prosthesis. The implant boundary is used to create a measurement boundary displaced from the implant boundary toward the bone producing a conformal region of interest. Matched histograms of bone density in a lateral and medial such region of interest may be displayed to evaluate symmetrical stress effects. Alternatively, bone density may be displayed plotted along an axis cutting across the medial and lateral sides. Fiducial points are identified from the bone and implant morphology to ensure that either display will have a repeatable reference and hence that such displays will be directly comparable to later and earlier displays thereby aiding in the detection of bone change.

### SUMMARY

This disclosure describes a variety of systems, devices, and techniques for providing patient analysis, preoperative planning, interoperative guidance, and/or training and education for revisional surgical joint repair procedures.

Consider a patient entering a healthcare facility, for example, at a beginning of a surgical lifecycle. The healthcare facility includes various imaging technologies for generating image data of the patient's anatomical regions including any implant component(s). After an examination of these images, professionals at the healthcare facility recommend a surgical procedure to replace an implant and some other follow up surgical procedure, which is referred to as a revision surgery. In one example, the healthcare facility recommends orthopedic revision surgery replace one or more implant components implanted into bone structure(s) around a joint (e.g., a scapula and/or a humerus around a shoulder joint). There are a number of reasons for performing the orthopedic revision surgery including an impaired implant and other impairments from a previous surgery, new disease(s) around the implant area, etc.

In example revision surgeries, once the implant is actually removed and another implant is to be inserted, there can be issues with knowing exactly where on a bone is an effective area to successfully install that new implant. One example technique described herein leverages various informational datasets, including bone density information, to mitigate these issues and improve upon surgical visualization and planning during the surgical lifecycle. Bone density information, in general, includes bone density measurements indicating bone qualities and/or characteristics for different areas of a bone structure. These bone qualities and/or characteristics allow surgical systems and surgeons to identify effective areas on the bone for removing a previous implant and/or implanting a replacement implant. The techniques described herein achieve a considerable degree of precision of where an implant is to be implanted with respect to the bone density measurements, enabling surgical tools and surgeons to identify within the above-mentioned identified effective areas specific locations for applying surgical instruments.

An example surgical system (e.g., a computing device/system having various hardware and/or software) includes various technologies to facilitate orthopedic revision surgeries by implementing one or more techniques for surgical planning and guidance as described herein. Visualization tools, one example technology of the example surgical system, may be configured to facilitate an orthopedic revision surgery by providing improved visualization of a region of an implant to be replaced (e.g., with another implant). Some example visualization tools include software application code that when executed on a hardware processor, enhance surgical planning and guidance as currently provided by conventional surgical technologies by enabling the improved visualization of the region of the implant to be replaced.

Utilizing at least one example technique described herein, the visualization tool of the example surgical system may segment image data to separate out an existing implant, particularly a metallic implant, reforming a shoulder joint formed by the scapula and the humerus. The image data generally refers to pixel data or voxel data (e.g., such as intensity data of a pixel or voxel). That image data may be manipulated in such a manner that the implant can be removed virtually and occluded regions, including areas of the bone structure around and underneath the implant are exposed in the manipulated image data. To effectuate the removal in one example, the surgical system generates from the manipulated image data a second set of image data depicting, in the areas of the region of the removed implant, one or more representations of the bone density measurements.

When generating the second set of image data for use in surgical planning before the revision surgery, surgical guidance during the revision surgery, and/or surgical review/recovery after the revision surgery, the example surgical system implementing the techniques described herein may combine bone density information with bone models by directing the visualization tool to modify image data for a region of a patient having an implant currently implanted, with the bone density information, on a bone structure of interest. One example surgical system replaces the image data for the region of the implant with a representation of the bone density information in the form of textual data annotating areas of the region with bone density measurements. Another example surgical system overlays the textual data representing the bone density information over the image data in the areas of the region of the implant, annotating areas of the region with the bone density measurements. Yet another example surgical system replaces the image data for the region of the implant with a graphical representation of the bone density information such that each bone density measurement is represented by a unique visual attribute (e.g., a unique color). In one example, data for the graphical representation may include intensity data for pixels or voxels that when rendered, produces specific colors or shades indicating different bone density measurements.

Example surgical systems may generate, as an example of the second set of image data, a bone density map depicting representations of different bone qualities/characteristics (e.g., impaired or "bad" bone and healthy or "good" bone) for the areas of the region of the removed implant. Each unique bone quality or characteristic refers to a possible classification by the computing device based upon the bone density information. To illustrate by way of example, if an area has a high bone density measurement (which is defined by being a value greater than a threshold value), that area has sufficient bone density and may be classified as healthy or "good" bone; whereas, an area with insufficient bone density is classified as impaired or "bad" bone for having a low bone density measurement (as defined by a value less than the same threshold value or another threshold value). The bone density map may identify, in the region of the removed implant, areas of "good" bone and "bad" bone classifications using a first color and a second color. As an alternative or in addition, the bone density map may depict textual data indicating which areas are classified as "good" bone and which areas are classified as "bad" bone.

According to an embodiment of the invention and as defined in claim 1, there is provided a system for modeling bone density information, the system comprising a memory configured to store a first set of patient-specific image data, the first set of patient-specific image data including image data for an implant implanted in a region of a patient; and processing circuitry configured to: segment the implant from the region in the first set of patient-specific image data; generate a second set of patient-specific image data based on removal of segments of the implant from the first set of patient-specific image data; generate the bone density information for the region based on the second set of patient-specific image data; identify areas in the second set of patient-specific image data based upon the bone density information; and output, the identified areas, to at least one display device. The processing circuitry is further configured to: output, to a subsystem of an orthopedic surgical system, a recommendation as to which of at least one of an implant type, a treatment type, or a surgical instrument to use for a revision surgery, the recommendation being based on the bone density information.

According to another embodiment of the invention and as defined in claim 11, there is provided a method for modeling bone density information that comprises: storing, in a memory, a first set of patient-specific image data, the first set of patientspecific image data including image data for an implant implanted in a region of a patient; segmenting the implant from the region in the first set of patient-specific image data; generating a second set of patient-specific image data based on removal of segments of the implant from the first set of patientspecific image data; generating the bone density information for the region based on the second set of patient-specific image data; identifying areas in the second set of patient-specific image data based upon the bone density information; and outputting, the identified areas, to at least one display device, wherein outputting the identified areas further comprises: outputting, to a subsystem of an orthopedic surgical system, a recommendation as to which of at least one of an implant type, a treatment type, or a surgical instrument to use for a revision surgery, the recommendation being based on the bone density information.

According to another embodiment of the invention and as defined in claim 14, there is provided a computer readable storage medium comprising instructions that, when executed by processing circuitry, causes the processing circuitry to perform the method defined in claim 11.

The details of various examples of the disclosure are set forth in the accompanying drawings and the description below. Various features, objects, and advantages will be apparent from the description, drawings, and claims.

### BRIEF DESCRIPTION OF THE DRAWINGS

FIG. 1 is a block diagram of an orthopedic surgical system, according to an example of this disclosure.
FIG. 2 is a block diagram of an orthopedic surgical system that includes a mixed reality (MR) system, according to an example of this disclosure.
FIG. 3 is a flowchart illustrating example phases of a surgical lifecycle.
FIG. 4 is a flowchart illustrating preoperative, intraoperative and postoperative workflows in support of an orthopedic surgical procedure.
FIG. 5 is a schematic representation of a visualization device for providing interoperative (MR) guidance using the MR system of FIG.2, according to an example of this disclosure.
FIGS. 6A and 6B are illustrations of example muscles and bones related to a shoulder of a patient.
FIG. 7 is a block diagram illustrating example components of a system configured to determine from patient-specific image data bone density and other information related to surgical revision, according to an example of this disclosure.
FIG. 8 is a flowchart illustrating an example procedure for modeling bone density information for a revision surgery, in accordance with a technique of this disclosure.
FIG. 9 is a flowchart illustrating an example procedure for modeling bone structure with an implant removed using patient-specific image data, in accordance with a technique of this disclosure.
FIG. 10A is a flowchart illustrating an example procedure for presenting a representation of a bone structure model with an implant removed, in accordance with one or more techniques of this disclosure.
FIG. 10B is a flowchart illustrating an example procedure for determining recommendations for surgical revision based on bone density, in accordance with one or more techniques of this disclosure.
FIG. 11 is a flowchart illustrating an example procedure for displaying bone density information, in accordance with one or more techniques of this disclosure.
FIG. 12 is a conceptual diagram of an example user interface that includes a humeral head and cutting plane.
FIG. 13 is a conceptual diagram of an example user interface that includes a humeral head and a representation of internal bone density.
FIG. 14 is a conceptual diagram of an example user interface that includes a humeral head and a representation of internal bone density associated with a type of humeral implant recommendation.
FIG. 15 is an illustration of a bone density map for use by an MR system when providing revision guidance for a shoulder replacement.
FIG. 16A is an illustration of a bone density map for a humerus with an implant.
FIG. 16B is an illustration of a bone density map for a humerus without an implant.
FIG. 17A is an illustration of a bone density map for a humerus with an implant.
FIG. 17B is an illustration of a bone density map for a humerus without an implant.

### DETAILED DESCRIPTION

This disclosure describes a variety of systems, devices, and techniques for providing patient analysis, preoperative planning, and/or training and education for surgical joint repair procedures. Orthopedic surgery can involve implanting one or more prosthetic devices to repair or replace a patient's damaged or diseased joint. Virtual surgical systems use image data of the diseased or damaged joint to generate an accurate three-dimensional bone model that can be viewed and manipulated preoperatively, for instance, by augmenting the bone model with bone density information. These surgical systems can enhance surgical outcomes by allowing the surgeon to simulate the surgery, select or design an implant that more closely matches the contours of the patient's actual bone, and select or design surgical instruments and guide tools that are adapted specifically for repairing the bone of a particular patient.

Similarly, these surgical systems can enhance surgical outcomes in orthopedic revision surgery where the implant is removed and then, replaced with a new implant and/or repaired with some treatment. These surgical systems can further enhance surgical outcomes by determining bone density information for areas of the patient's bone from where the implant is to be removed and replaced. Based upon the bone density information, these technologies may determine qualities and/or characteristics for the areas of the patient's actual bone proximate to the implant including occluded areas by the implant and then, by outputting image data depicting a distribution of different bone qualities and/or characteristics in at least a portion of the patient's actual bone where each bone quality or characteristic of interest corresponds to a unique representation, the surgical systems may enhance surgical outcomes. For example, different sets of visual attributes (e.g., color, shade, etc.) can be used to create distinguishing features for each bone quality or characteristic of interest, enabling quick identification by a surgeon.

These surgical systems can be used to generate a preoperative surgical plan, complete with an implant and surgical instruments that are selected or manufactured for the individual patient. In some example, the surgeon may utilize these surgical systems for viewing image data differentiating areas of the patient's bone where there is sufficient bone density from areas where there is insufficient bone density. These surgical systems may recommend a specific treatment for the individual patient. These surgical systems may rely on bone models for the patient for determining types of procedures and/or specific implants for the individual patient. However, bone density information cannot be derived from image data for certain regions of the patient, such as regions covered by the implant. Without this image data for the bone structures of the patient, the planning tools and the clinician may determine certain aspects of the revision surgery or implant without the benefit of how the patient's bone structures may affect the function of the joint and the joint post-revision surgery.

These surgical systems can be used to provide through one or more MR systems intraoperative revision guidance in which a surgeon is directed, in real-time, to locations for applying a surgical instrument in furtherance of a treatment type and implanting an implant to replace a current implant. These locations are typically occluded by the implant and without a proper image data analysis for these locations, the planning tools and the clinician can direct the surgeon through the revision surgery but without knowing which areas have sufficient bone density and which areas do not have sufficient bone density. For at least this reason, the surgeon may mistakenly injure the patient during a surgical procedure and/or apply an improper implant component that is likely to fail, for example, for applying the surgical instrument to one or more areas of insufficient or low-quality bone density.

When a surgical procedure or implant component fails, a patient with one or more implant components experiences a trauma or another complication such as infection, a disease state progressing further to a point that an implant operation fails, or an existing implant component otherwise failing or stopping working properly. The presence of an existing implant component may correlate with a patient also having bone fractures or fragments or an otherwise deteriorated bone condition. Obtaining good imaging, segmentation, and modeling of a bone with an implant can be of particular importance in pre-operative and intra-operative planning for surgical revision procedures and post-operative training of models. As an example, revision surgery results and plans can be used as historical data for the pre-operative and intra-operative planning for future surgical revision procedures.

This disclosure describes techniques for preparing a surgical revision procedure plan using bone density information corresponding to image data in which an implant has been removed by way of an imaging process that removes the implant from a first set of patient-specific image data. The result of the removal is a second set of patient-specific image data with the implant removed. In some examples, the second set of patient-specific image data includes intensity values associated with presenting the bone density information for the surrounding and/or exposed areas of the bone structure. As an alternative, the imaging process removes the implant by segmenting image data representative of an orthopedic joint in which one or more implant components have been placed, identifying a region containing at least one implant component, and overlaying the bone density information over the region such that bone density values appear as numerical values over corresponding pixels or voxels (or groups of pixels or voxels) in the region.

In some examples, the techniques may enable a computing device to provide intra-operative surgical guidance to a surgeon or surgical team to perform a surgical revision procedure based on at least some of the above-mentioned bone density information for a patient. Thus, the techniques of this disclosure may improve existing computing devices by enabling computing devices to support better pre-operative planning and/or intra-operative guidance for surgical revision procedures. This better surgical planning and/or guidance may take the form of improved segmentation of a joint with an existing implant but may also take the form of more reliable surgical recommendations, such as automated recommendations to a surgeon related to procedure types and implant types for surgical revision to replace such existing implant components.

Shoulder replacement surgery is described as one example herein. However, the systems, devices, and techniques described herein may be employed to analyze other anatomical structures or groups of structures of a patient, determine a type of treatment for other joints of the patient (e.g., elbow, hip, knee, etc.), or select a certain type of implant for the particular anatomical condition of the patient. The techniques described herein for removing implant component from patient-specific image data to expose bone structure(s) around the shoulder can be applied to other bone structures. In addition, the techniques described herein for combining bone density information with the patient-specific image data such that the exposed bone structure(s) in the patient-specific image data includes areas annotated with values indicating bone densities may be employed to other types of helpful information and/or to other structures, such as soft-tissue structures, in other examples.

In some examples, systems, devices, and methods may employ a mixed reality (MR) visualization system to assist with creation, implementation, verification, and/or modification of a surgical revision procedure plan (which is a type of surgical plan) before and during a surgical procedure, such as those processes associated with determining which type of treatment to provide to the patient (e.g., a joint replacement surgery such as shoulder replacement). Because MR, or in some instances VR, may be used to interact with the surgical plan, this disclosure may also refer to the surgical plan as a "virtual" surgical plan. Visualization tools other than or in addition to mixed reality visualization systems may be used in accordance with techniques of this disclosure.

A surgical plan or recommendation, e.g., as generated by the BLUEPRINT ^{™} system, available from Wright Medical, Inc., or another surgical planning platform, may include information defining a variety of features of a surgical procedure, such as suggested types of surgical treatment (e.g., anatomical or reverse shoulder surgery) features of particular surgical procedure steps to be performed on a patient by a surgeon according to the surgical plan including, for example, bone or tissue preparation steps and/or steps for selection, modification and/or placement of implant components. Such information may include, in various examples, dimensions, shapes, angles, surface contours, and/or orientations of implant components to be selected or modified by surgeons, dimensions, shapes, angles, surface contours and/or orientations to be defined in bone or soft tissue by the surgeon in bone or tissue preparation steps, and/or positions, axes, planes, angle and/or entry points defining placement of implant components by the surgeon relative to patient bone or other tissue. Information such as dimensions, shapes, angles, surface contours, and/or orientations of anatomical features of the patient may be derived from imaging (e.g., x-ray, CT, MRI, ultrasound or other images), direct observation, or other techniques.

Some visualization tools utilize patient image data to generate three-dimensional models of bone contours to facilitate preoperative planning for joint repairs and replacements. These tools may allow surgeons to design and/or select surgical guides and implant components that closely match the patient's anatomy. These tools can improve surgical outcomes by customizing a surgical plan for each patient. An example of such a visualization tool for shoulder repairs is the BLUEPRINT ^{™} system identified above. The BLUEPRINT ^{™} system provides the surgeon with two-dimensional planar views of the bone repair region as well as a three-dimensional virtual model of the repair region. The surgeon can use the BLUEPRINT ^{™} system to select, design or modify appropriate implant components, determine how best to position and orient the implant components and how to shape the surface of the bone to receive the components, and design, select or modify surgical guide tool(s) or instruments to carry out the surgical plan. The information generated by the BLUEPRINT ^{™} system is compiled in a preoperative surgical plan for the patient that is stored in a database at an appropriate location (e.g., on a server in a wide area network, a local area network, or a global network) where it can be accessed by the surgeon or other care provider, including before and during the actual surgery.

To illustrate by way of example, via a display device coupled to the example computing device/system, an example surgical may present to a surgeon, doctor, or other user the bone density map depicting bone structures around the patient's shoulder joint. In the bone density map, areas on these bone structures, including the scapula, the clavicle, the glenoid, the humerus, and the like, are annotated with one or more bone density classifications. In this manner, the bone density map serves as a representation of the bone density information for the bone structures around the patient's joint (e.g., shoulder joint) and that representation differentiates between areas of different bone qualities or characteristics, allowing for quick identification of areas of sufficient bone density.

A user interface operating on the display device may present to a surgeon the bone density map as a preoperative surgical plan through one or more virtual planning subsystems of the surgical system. An example virtual planning subsystem generates the preoperative surgical plan to identify, on the bone structures around the patient's joint, locations onto a which a surgical instrument is to be applied for safely and effectively removing an implant and/or implanting a new implant during revision surgery. The example virtual planning subsystem may determine that these locations have sufficient bone density to accommodate the application of the surgical instrument as well as the removal plus replacement of the currently implanted implant.

Another user interface operating on the display device may present the representation to a surgeon as interoperative surgical guidance through one or more mixed reality (MR) systems. An example MR system may augment a view into the patient's shoulder with bone density information (if not already done so). The example MR system may include a graphical representation of a surgical instrument (or a pointer) at a location into which the surgeon is to apply the surgical instrument. An alternative MR system may present a visualization of a revision plan developed during planning. Another user interface may present the representation to the surgeon during preoperative planning along with recommended surgical instruments and replacement implant components. The bone density information may be used for surgical planning, such as in determining replacement implant types, treatment types, surgical instruments, and/or the like to recommend for the revision surgery.

In some examples, the computing device provides intraoperative mixed reality (MR) guidance with presentation of patient-specific image data for a joint before or after an imaging process removed the implant. Using a visualization device, a surgeon may view a scapula with or without the implant and see both views simultaneously or as alternative views on a same display area. In some example, the computing device presents bone density information for any bone(s) around the joint into which the implant was implanted and/or to be removed in a revision surgery. Using a visualization device, a surgeon may view a bone density map indicating bone qualities/characteristics of a surface of a bone structure before or after implant removal. The bone density map may delicate areas of the bone where bone density/quality is impaired (e.g., lower in bone density than desired) so the surgeon can avoid these areas.

Certain examples of this disclosure are described with reference to the accompanying drawings, wherein like reference numerals denote like elements. It should be understood, however, that the accompanying drawings illustrate only the various implementations described herein and are not meant to limit the scope of various technologies described herein. The drawings show and describe various examples of this disclosure.

In the following description, numerous details are set forth to provide an understanding of the present disclosure. However, it will be understood by those skilled in the art that one or more aspects of the present disclosure may be practiced without these details and that numerous variations or modifications from the described examples may be possible.

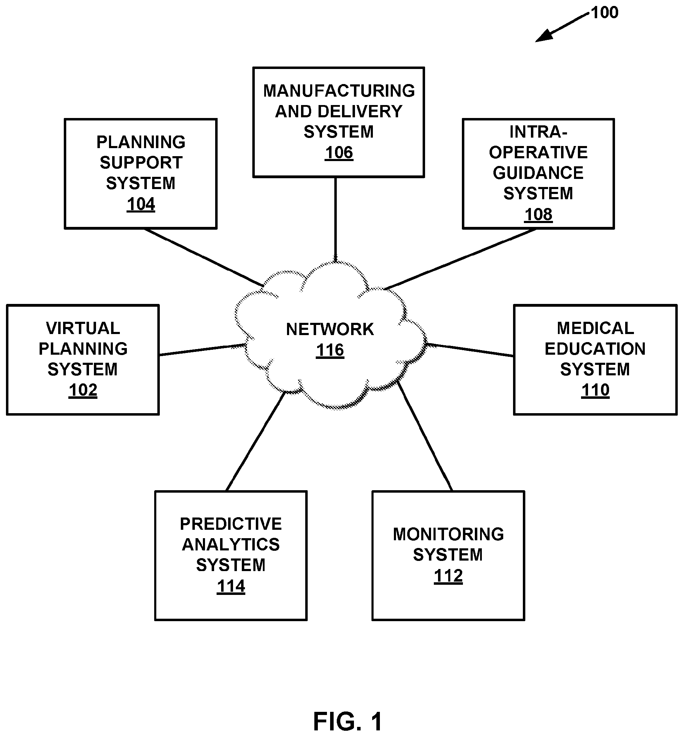

FIG. 1 is a block diagram of an orthopedic surgical system 100 according to an example of this disclosure. Orthopedic surgical system 100 is an example surgical system as described herein. Orthopedic surgical system 100 includes a set of subsystems. In the example of FIG. 1, the subsystems include a virtual planning system 102, a planning support system 104, a manufacturing and delivery system 106, an intraoperative guidance system 108, a medical education system 110, a monitoring system 112, a predictive analytics system 114, and a communications network 116. In other examples, orthopedic surgical system 100 may include more, fewer, or different subsystems. For example, orthopedic surgical system 100 may omit medical education system 110, monitor system 112, predictive analytics system 114, and/or other subsystems. In some examples, orthopedic surgical system 100 may be used for surgical tracking, in which case orthopedic surgical system 100 may be referred to as a surgical tracking system. In other cases, orthopedic surgical system 100 may be generally referred to as a medical device system.

Users of orthopedic surgical system 100 may use virtual planning system 102 to plan orthopedic surgeries. For example, virtual planning system 102 and/or another surgical planning system may analyze patient-specific image data and determine suggested surgical treatments and/or surgical instruments based on bone density information, as discussed herein. The patient image data, as described herein, may result from an imaging process that removes image data of an implant. Users of orthopedic surgical system 100 may use planning support system 104 to review surgical plans generated using orthopedic surgical system 100. Manufacturing and delivery system 106 may assist with the manufacture and delivery of items needed to perform orthopedic surgeries. Intraoperative guidance system 108 provides guidance to assist users of orthopedic surgical system 100 in performing orthopedic surgeries. Medical education system 110 may assist with the education of users, such as healthcare professionals, patients, and other types of individuals. Pre- and postoperative monitoring system 112 may assist with monitoring patients before and after the patients undergo surgery. Predictive analytics system 114 may assist healthcare professionals with various types of predictions. For example, predictive analytics system 114 may apply artificial intelligence techniques to determine a classification of a condition of an orthopedic joint, e.g., a diagnosis, determine which type of surgery to perform on a patient and/or which type of implant to be used in the procedure, determine types of items that may be needed during the surgery, and so on.

The subsystems of orthopedic surgical system 100 (e.g., virtual planning system 102, planning support system 104, manufacturing and delivery system 106, intraoperative guidance system 108, medical education system 110, pre- and postoperative monitoring system 112, and predictive analytics system 114) may include various systems. The systems in the subsystems of orthopedic surgical system 100 may include various types of computing systems, computing devices, including server computers, personal computers, tablet computers, smartphones, display devices, Internet of Things (IoT) devices, visualization devices (e.g., mixed reality (MR) visualization devices, virtual reality (VR) visualization devices, holographic projectors, or other devices for presenting extended reality (XR) visualizations), surgical tools, and so on. A holographic projector, in some examples, may project a hologram for general viewing by multiple users or a single user without a headset, rather than viewing only by a user wearing a headset. For example, virtual planning system 102 may include a MR visualization device and one or more server devices, planning support system 104 may include one or more personal computers and one or more server devices, and so on. A computing system is a set of one or more computing devices and/or systems configured to operate as a system. In some examples, one or more devices may be shared between the two or more of the subsystems of orthopedic surgical system 100. For instance, in the previous examples, virtual planning system 102 and planning support system 104 may include the same server devices.

Example MR visualization devices include head-mounted display devices such as the Microsoft HOLOLENS ^{™} headset, available from Microsoft Corporation of Redmond, Washington, which includes see-through holographic lenses, sometimes referred to as waveguides, that permit a user to view real-world objects through the lens and concurrently view projected 3D holographic objects. The Microsoft HOLOLENS ^{™} headset, or similar waveguide-based visualization devices, are examples of an MR visualization device that may be used in accordance with some examples of this disclosure. Some holographic lenses may present holographic objects with some degree of transparency through see-through holographic lenses so that the user views real-world objects and virtual, holographic objects. In some examples, some holographic lenses may, at times, completely prevent the user from viewing real-world objects and instead may allow the user to view entirely virtual environments. The term mixed reality may also encompass scenarios where one or more users are able to perceive one or more virtual objects generated by holographic projection. In other words, "mixed reality" may encompass the case where a holographic projector generates holograms of elements that appear to a user to be present in the user's actual physical environment. Although MR visualization devices are described as one example herein, display screens such as cathode ray tube (CRT) displays, liquid crystal displays (LCDs), and light emitting diode (LED) displays may be used to present any aspect of the information described herein in other examples.

In the example of FIG. 1, the devices included in the subsystems of orthopedic surgical system 100 may communicate using communication network 116. Communication network 116 may include various types of communication networks including one or more wide-area networks, such as the Internet, local area networks, and so on. In some examples, communication network 116 may include wired and/or wireless communication links.

As described herein, the patient image data being used by orthopedic surgical system 100 undergoes an imaging technique modifying a first set of patient-specific image data such that the patient's implant is removed from a corresponding region of the image. A subsystem of orthopedic surgical system 100, such as virtual planning system 102, may execute the imaging process. By applying a segmentation process as part of the imaging technique, orthopedic surgical system 100 segments a region of the first set of patient-specific data depicting an implant and then, removes the segmented region to expose bone structure occluded by the implant. By outputting a final representation of the exposed bone structure, orthopedic surgical system 100 generates a surgical plan for a revision surgery that is performed to replace or compensate for a failed implant (e.g., a shoulder replacement).

By analyzing the exposed bone structure of the occluded region, orthopedic surgical system 100 provides information to the benefit of the patient undergoing the revision surgery. In general, the information identifies points or areas of the exposed bone structure having a certain bone quality/characteristic. For instance, orthopedic surgical system 100 may implement a bone density metric to determine a bone density and to further determine a bone quality/characteristic for one or more areas of the patient's bone with the implant removed. Orthopedic surgical system 100 may define one example bone quality/characteristic in terms of a classification system that indicates cortical or "good" bone, cancellous or "bad" bone, and/or one or more classifications between "good" bone or "bad" bone (e.g., "satisfactory" bone, "impaired" bone, and/or the like). This example bone quality/characteristic classification system may be measured using numerical values, such as a range of discrete or continuous values indicating a bone quality/characteristic between "good" bone or "bad" bone. An example numerical value represents a bone density value corresponding to a portion (e.g., a specific point, area, or region) of the patient's bone; in some examples of the above-mentioned classification system, "good" bone and "bad" bone refer to non-overlapping areas of the patient's bone corresponding to bone density values that are greater than a first threshold (e.g., a high value) and less than a second threshold (e.g., a low value), respectively.

Each respective area may be depicted with a same color and an overload bone density value that corresponds to that area. In another example, each respective area may be depicted with a color representative of that area's classification or bone density value. Areas of "good" bone and "bad" bone may be depicted using black and white colors, respectively, such that gray-scale values may represent intermediate bone density classifications between "good" bone and "bad" bone. In one example, orthopedic surgical system 100 identifies an area as implant position and computes a bone quality/characteristic of that implant position. The implant position refers to a position on the bone where an implant is inserted. Orthopedic surgical system 100 may determine bone density values for areas around the inserted implant parts, classify the bone density values, and output a bone quality/characteristic to a surgeon. As an example, orthopedic surgical system 100 may output information indicating that the implant position is stable, weak, sufficient, insufficient, and/or like.

In a visualization device (e.g., a MR visualization device), respective "good" and "bad" areas of the patient's bone are displayed in any manner that allows a surgeon to distinguish between them when viewing image data of the patient's bone. The visualization device may employ any suitable imaging technique to properly display the patient's bone while differentiating the respective "good" and "bad" areas of the patient's bone. The surgeon and the patient (e.g., during revision surgery) benefit from having this image data available because, for instance, the surgeon can easily identify areas into which an implant is to be removed/implanted and avoid areas that may harm the patient and/or impede the surgery. To further illustrate the benefit by way of an example, the surgeon may use the visualization device for pre-planning and guidance by avoiding areas that are depicted as having "bad" bone and focus on areas that are depicted as having "good" bone. By considering only the "good" bone areas as candidates for applying the surgical instruments and implanting the implant, the surgeon reduces risk and improves upon the effectiveness of the revision surgery.

Surgical revision plans generated by orthopedic surgical system 100 incorporates such information, for example, to identify in the patient points or areas (in three-dimensional space) to which a surgical profession applies a surgical instrument. With respect to the application of the surgical instrument in the revision plan, there are a number of example use cases involving scalpels, drills, and/or the like of which at least some are directed to precise patent points or areas as opposed to general regions of the patient. Enabled in part because of the segmentation and simulated removal of an implant, some revision plans pinpoint exactly where to drill to safely remove an old implant or implant a new implant based upon locations of "good" bone and "bad" bone in the patient.

To illustrate by way of example, in an example revision plan for a shoulder implant, a surgeon is directed to operate a specific drill in areas having "good" bone while avoiding areas having "bad" bone. As described herein, the segmentation and simulated removal exposes addition regions (e.g., occluded regions) to analyze for bone density information and this new information provides accuracy and precision in the identification of the locations of "good" bone and "bad" bone in the patient. With CT technology and the techniques described herein for the patient-specific image data, orthopedic surgical system 100 achieves an exceptional level of precision for the revision plans. In other examples, the revision plans generated by orthopedic surgical system 100 incorporates the above-mentioned bone density information, for example, to identify in the patient points or areas (in three-dimensional space) to which a surgical profession applies a surgical instrument, to determine a suitable replacement implant or, as an alternative, a compensatory medical device to place into the patient, and so forth.

Having precise bone density information at an enhanced resolution for areas surrounding an implant and for occluded areas underneath the implant, orthopedic surgical system 100 may generate revision plans to improve revision surgeries and such improvements may benefit a preoperative stage, an intraoperative stage, and/or a postoperative stage. For example, a subsystem of orthopedic surgical system 100 may utilize a head-mounted device to display a view of a patient during a revision surgery and augment that view with bone density information for (e.g., overlaying) areas of the patient's bone structure.

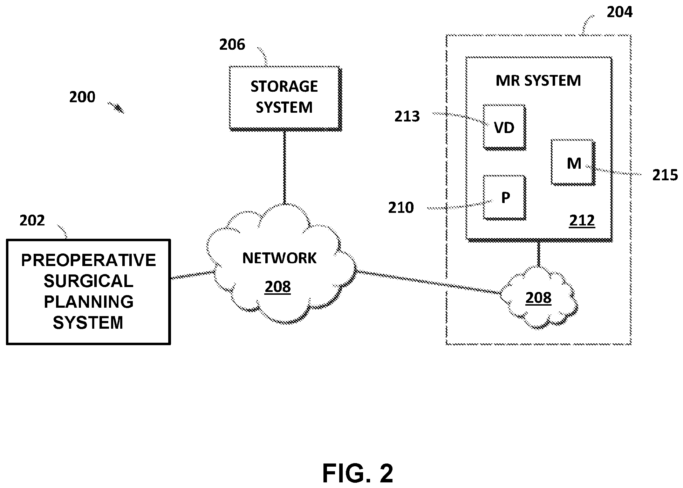

Many variations of orthopedic surgical system 100 are possible. Such variations may include more or fewer subsystems than the version of orthopedic surgical system 100 shown in FIG. 1. For example, FIG. 2 is a block diagram of an orthopedic surgical system 200 that includes one or more mixed reality (MR) systems, according to an example of this disclosure. Orthopedic surgical system 200 may be used for creating, verifying, updating, modifying and/or implementing a surgical plan. In some examples, the surgical plan can be created preoperatively, such as by using a virtual surgical planning system (e.g., the BLUEPRINT ^{™} system), and then verified, modified, updated, and viewed intraoperatively, e.g., using MR visualization or other visualization of the surgical plan. In other examples, orthopedic surgical system 200 can be used to create the surgical plan immediately prior to surgery or intraoperatively, as needed. In some examples, orthopedic surgical system 200 may be used for surgical tracking, in which case orthopedic surgical system 200 may be referred to as a surgical tracking system. In other cases, orthopedic surgical system 200 may be generally referred to as a medical device system.

In the example of FIG. 2, orthopedic surgical system 200 includes a preoperative surgical planning system 202, a healthcare facility 204 (e.g., a surgical center or hospital), a storage system 206 and a network 208 that allows a user at healthcare facility 204 to access stored patient information, such as medical history, image data corresponding to the damaged joint or bone and various parameters corresponding to a surgical plan that has been created preoperatively (as examples). Preoperative surgical planning system 202 may be equivalent to virtual planning system 102 of FIG. 1 and, in some examples, may generally correspond to a virtual planning system similar or identical to the BLUEPRINT ^{™} system.

In the example of FIG. 2, healthcare facility 204 includes a mixed reality (MR) system 212. In some examples of this disclosure, MR system 212 includes one or more processing device(s) (P) 210 to provide functionalities such as presentation of visual information to a user that relates to preoperative planning, intraoperative guidance, or even postoperative review and follow up. Processing device(s) 210 may also be referred to as processor(s). In addition, one or more users of MR system 212 (e.g., a surgeon, nurse, or other care provider) can use processing device(s) (P) 210 to generate a request for a particular surgical plan or other patient information that is transmitted to storage system 206 via network 208. In response, storage system 206 returns the requested patient information to MR system 212. In some examples, the users can use other processing device(s) to request and receive information, such as one or more processing devices that are part of MR system 212, but not part of any visualization device, or one or more processing devices that are part of a visualization device (e.g., visualization device 213) of MR system 212, or a combination of one or more processing devices that are part of MR system 212, but not part of any visualization device, and one or more processing devices that are part of a visualization device (e.g., visualization device 213) that is part of MR system 212. In other words, an example MR visualization device such as the Microsoft HOLOLENS^{™} device may include all of the components of MR system 212, or utilize one or more external processors and/or memory to perform some or all processing functionality necessary for a passive visualization device 213.

In some examples, multiple users can simultaneously use MR system 212. For example, MR system 212 can be used in a spectator mode in which multiple users each use their own visualization devices so that the users can view the same information at the same time and from the same point of view. In some examples, MR system 212 may be used in a mode in which multiple users each use their own visualization devices so that the users can view the same information from different points of view.

In some examples, processing device(s) 210 can provide a user interface to display data and receive input from users at healthcare facility 204. Processing device(s) 210 may be configured to control visualization device 213 to present a user interface. Furthermore, processing device(s) 210 may be configured to control visualization device 213 (e.g., one or more optical waveguides such as a holographic lens) to present virtual images, such as 3D virtual models, 2D images, surgery plan information, and so on. Processing device(s) 210 can include a variety of different processing or computing devices, such as servers, desktop computers, laptop computers, tablets, mobile phones and other electronic computing devices, or processors within such devices. In some examples, one or more of processing device(s) 210 can be located remote from healthcare facility 204. In some examples, processing device(s) 210 reside within visualization device 213. In some examples, at least one of processing device(s) 210 is external to visualization device 213. In some examples, one or more processing device(s) 210 reside within visualization device 213 and one or more of processing device(s) 210 are external to visualization device 213.

In the example of FIG. 2, MR system 212 also includes one or more memory or storage device(s) (M) 215 for storing data and instructions of software that can be executed by processing device(s) 210. The instructions of software can correspond to the functionality of MR system 212 described herein. In some examples, the functionalities of a virtual surgical planning application, such as the BLUEPRINT ^{™} system, can also be stored and executed by processing device(s) 210 in conjunction with memory storage device(s) (M) 215. For instance, memory or storage system 215 may be configured to store data corresponding to at least a portion of a virtual surgical plan. In some examples, storage system 206 may be configured to store data corresponding to at least a portion of a virtual surgical plan. In some examples, memory or storage device(s) (M) 215 reside within visualization device 213. In some examples, memory or storage device(s) (M) 215 are external to visualization device 213. In some examples, memory or storage device(s) (M) 215 include a combination of one or more memory or storage devices within visualization device 213 and one or more memory or storage devices external to the visualization device.

Network 208 may be equivalent to network 116. Network 208 can include one or more wide area networks, local area networks, and/or global networks (e.g., the Internet) that connect preoperative surgical planning system 202 and MR system 212 to storage system 206. Storage system 206 can include one or more databases that can contain patient information, medical information, patient image data, and parameters that define the surgical plans. For example, medical images of the patient's diseased or damaged bone and/or soft tissue typically are generated preoperatively in preparation for an orthopedic surgical procedure. The medical images can include images of the relevant bone(s) and/or soft tissue taken along the sagittal plane and the coronal plane of the patient's body. The medical images can include X-ray images, magnetic resonance imaging (MRI) images, computed tomography (CT) images, ultrasound images, and/or any other type of 2D or 3D image that provides information about the relevant surgical area. Storage system 206 also can include data identifying the implant components selected for a particular patient (e.g., type, size, etc.), surgical guides selected for a particular patient, and details of the surgical procedure, such as entry points, cutting planes, drilling axes, reaming depths, etc. Storage system 206 can be a cloud-based storage system (as shown) or can be located at healthcare facility 204 or at the location of preoperative surgical planning system 202 or can be part of MR system 212 or visualization device (VD) 213, as examples.

MR system 212 can be used by a surgeon before (e.g., preoperatively) or during the surgical procedure (e.g., intraoperatively) to create, review, verify, update, modify and/or implement a surgical plan. In some examples, MR system 212 may also be used after the surgical procedure (e.g., postoperatively) to review the results of the surgical procedure, assess whether revisions are required, or perform other postoperative tasks. In this manner, MR system 12 may enable the user to see real-world scenes such as anatomical objects in addition to virtual imagery (e.g., virtual glenoid or humerus images, guidance images, or other text or images) placed at that real-world scene. To that end, MR system 212 may include a visualization device 213 that may be worn by the surgeon and (as will be explained in further detail below) is operable to display a variety of types of information, including a 3D virtual image of the patient's diseased, damaged, or postsurgical joint and details of the surgical plan, such as images of bone and/or soft tissue of the patient derived from patient image data, generated models of bone, a 3D virtual image of the prosthetic implant components selected for the surgical plan, 3D virtual images of entry points for positioning the prosthetic components, alignment axes and cutting planes for aligning cutting or reaming tools to shape the bone surfaces, or drilling tools to define one or more holes in the bone surfaces, in the surgical procedure to properly orient and position the prosthetic components, surgical guides and instruments and their placement on the damaged joint, and any other information that may be useful to the surgeon to implement the surgical plan. MR system 212 can generate images of this information that are perceptible to the user of the visualization device 213 before and/or during the surgical procedure.

In some examples, MR system 212 includes multiple visualization devices (e.g., multiple instances of visualization device 213) so that multiple users can simultaneously see the same images and share the same 3D scene. In some such examples, one of the visualization devices can be designated as the master device and the other visualization devices can be designated as observers or spectators. Any observer device can be re-designated as the master device at any time, as may be desired by the users of MR system 212.

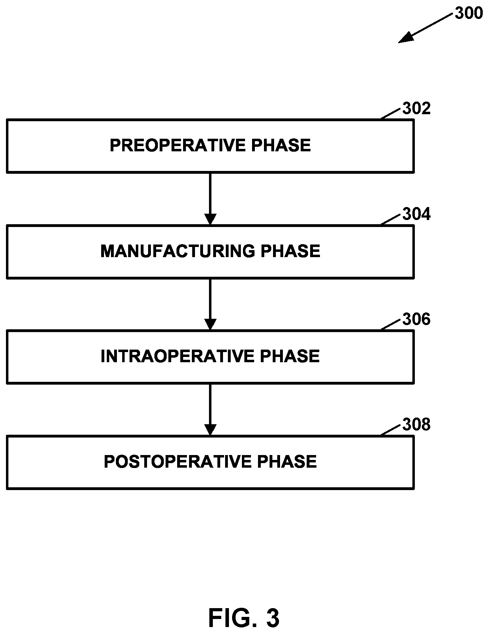

FIG. 3 is a flowchart illustrating example phases of a surgical lifecycle 300. In the example of FIG. 3, surgical lifecycle 300 begins with a preoperative phase (302). During the preoperative phase, a surgical plan is developed. The preoperative phase is followed by a manufacturing and delivery phase (304). During the manufacturing and delivery phase, patient-specific items, such as parts and equipment, needed for executing the surgical plan are manufactured and delivered to a surgical site. In some examples, it is unnecessary to manufacture patient-specific items in order to execute the surgical plan. An intraoperative phase follows the manufacturing and delivery phase (306). The surgical plan is executed during the intraoperative phase. In other words, one or more persons perform the surgery on the patient during the intraoperative phase. The intraoperative phase is followed by the postoperative phase (308). The postoperative phase includes activities occurring after the surgical plan is complete. For example, the patient may be monitored during the postoperative phase for complications.

As described in this disclosure, orthopedic surgical system 100 (FIG. 1) may be used in one or more of preoperative phase 302, the manufacturing and delivery phase 304, the intraoperative phase 306, and the postoperative phase 308. For example, virtual planning system 102 and planning support system 104 may be used in preoperative phase 302. In some examples, preoperative phase 302 may include the system analyzing patient image data, modeling bone, and/or determining or recommending a type of surgical treatment based on the condition of the patient. Manufacturing and delivery system 106 may be used in the manufacturing and delivery phase 304. Intraoperative guidance system 108 may be used in intraoperative phase 306. Some of the systems of FIG. 1 may be used in multiple phases of FIG. 3. For example, medical education system 110 may be used in one or more of preoperative phase 302, intraoperative phase 306, and postoperative phase 308; pre- and postoperative monitoring system 112 may be used in preoperative phase 302 and postoperative phase 308. Predictive analytics system 114 may be used in preoperative phase 302 and postoperative phase 308. Various workflows may exist within the surgical process of FIG. 3. For example, different workflows within the surgical process of FIG. 3 may be appropriate for different types of surgeries.

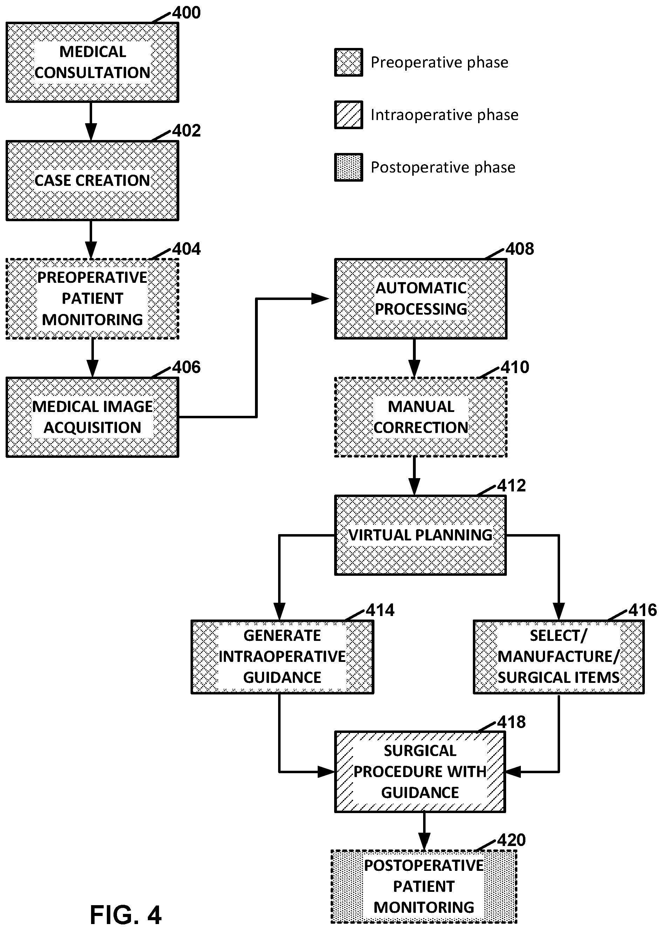

FIG. 4 is a flowchart illustrating example preoperative, intraoperative and postoperative workflows in support of an orthopedic surgical procedure. In the example of FIG. 4, the surgical process begins with a medical consultation (400). During the medical consultation (400), a healthcare professional evaluates a medical condition of a patient. For instance, the healthcare professional may consult the patient with respect to the patient's symptoms. During the medical consultation (400), the healthcare professional may also discuss various treatment options with the patient. For instance, the healthcare professional may describe one or more different surgeries to address the patient's symptoms.

Furthermore, the example of FIG. 4 includes a case creation step (402). In other examples, the case creation step occurs before the medical consultation step. During the case creation step, the medical professional or other user establishes an electronic case file for the patient. The electronic case file for the patient may include information related to the patient, such as data regarding the patient's symptoms, patient range of motion observations, data regarding a surgical plan for the patient, medical images of the patients, notes regarding the patient, billing information regarding the patient, and so on.

The example of FIG. 4 includes a preoperative patient monitoring phase (404). During the preoperative patient monitoring phase, the patient's symptoms may be monitored. For example, the patient may be suffering from pain associated with arthritis in the patient's shoulder. In this example, the patient's symptoms may not yet rise to the level of requiring an arthroplasty to replace the patient's shoulder. However, arthritis typically worsens over time. Accordingly, the patient's symptoms may be monitored to determine whether the time has come to perform a surgery on the patient's shoulder. Observations from the preoperative patient monitoring phase may be stored in the electronic case file for the patient. In some examples, predictive analytics system 114 may be used to predict when the patient may need surgery, to predict a course of treatment to delay or avoid surgery or make other predictions with respect to the patient's health.

Additionally, in the example of FIG. 4, a medical image acquisition step occurs during the preoperative phase (406). During the image acquisition step, medical images of the patient are generated. The medical images for a specific patient may be generated in a variety of ways. For instance, the images may be generated using a Computed Tomography (CT) process, a Magnetic Resonance Imaging (MRI) process, an ultrasound process, or another imaging process. The medical images generated during the image acquisition step include images of an anatomy of interest of the specific patient. For instance, if the patient's symptoms involve the patient's shoulder, medical images of the patient's shoulder may be generated. The medical images may be added to the patient's electronic case file. Healthcare professionals may be able to use the medical images in one or more of the preoperative, intraoperative, and postoperative phases.

Furthermore, in the example of FIG. 4, an automatic processing step may occur (408). During the automatic processing step, virtual planning system 102 (FIG. 1) may automatically develop a preliminary surgical plan for the patient. For example, virtual planning system 102 may generate a model, or representations of bone of the patient. If the patient's bone has an implant, virtual planning system 102 may segment the implant and remove the segmented implant from the above-mentioned patient-specific images. Based on these representations, virtual planning system 102 may determine bone density information including bone density classifications such as "good" bone and "bad" bone. Virtual planning system 102 may further transform the above-mentioned patient-specific images by generating image data depicting the model of the patient's bone with the implant removed and with bone density information, particularly in areas where the implant was implanted. Virtual planning system 102 may determine what types of treatment should be performed (e.g., whether a shoulder replacement should be an anatomical replacement or a reverse replacement) based on these qualities/characteristics. In some examples of this disclosure, virtual planning system 102 may use machine learning techniques to develop the preliminary surgical plan based on information in the patient's virtual case file.

The example of FIG. 4 also includes a manual correction step (410). During the manual correction step, one or more human users may check and correct the determinations made during the automatic processing step. In some examples of this disclosure, one or more users may use mixed reality or virtual reality visualization devices during the manual correction step. In some examples, changes made during the manual correction step may be used as training data to refine the machine learning techniques applied by virtual planning system 102 during the automatic processing step.

A virtual planning step (412) may follow the manual correction step in FIG. 4. During the virtual planning step, a healthcare professional may develop a surgical plan for the patient. In some examples of this disclosure, one or more users may use mixed reality or virtual reality visualization devices during development of the surgical plan for the patient.

Furthermore, in the example of FIG. 4, intraoperative guidance may be generated (414). The intraoperative guidance may include guidance to a surgeon on how to execute the surgical plan. In some examples of this disclosure, virtual planning system 102 may generate at least part of the intraoperative guidance. In some examples, the surgeon or other user(s) may contribute to the intraoperative guidance.

Additionally, in the example of FIG. 4, a step of selecting and manufacturing surgical items is performed (416). During the step of selecting and manufacturing surgical items, manufacturing and delivery system 106 (FIG. 1) may manufacture surgical items for use during the surgery described by the surgical plan. For example, the surgical items may include surgical implants, surgical tools, and other items required to perform the surgery described by the surgical plan.

In the example of FIG. 4, a surgical procedure may be performed with guidance from intraoperative guidance system 108 (FIG. 1) (418). For example, a surgeon may perform the surgery while wearing a head-mounted MR visualization device of intraoperative system 108 that presents guidance information to the surgeon. The guidance information may help guide the surgeon through the surgery, providing guidance for various steps in a surgical workflow, including sequence of steps, details of individual steps, and tool or implant selection, implant placement and position, and bone surface preparation for various steps in the surgical procedure workflow.

Postoperative patient monitoring may occur after completion of the surgical procedure (420). During the postoperative patient monitoring step, healthcare outcomes of the patient may be monitored. Healthcare outcomes may include relief from symptoms, ranges of motion, complications, performance of implanted surgical items, and so on. Pre- and postoperative monitoring system 112 (FIG. 1) may assist in the postoperative patient monitoring step.

The medical consultation, case creation, preoperative patient monitoring, image acquisition, automatic processing, manual correction, and virtual planning steps of FIG. 4 are part of preoperative phase 302 of FIG. 3. The surgical procedures with guidance steps of FIG. 4 is part of intraoperative phase 306 of FIG. 3. The postoperative patient monitoring step of FIG. 4 is part of postoperative phase 308 of FIG. 3.

As mentioned above, one or more of the subsystems of orthopedic surgical system 100 may include one or more mixed reality (MR) systems, such as MR system 212 (FIG. 2). Each MR system may include a visualization device. For instance, in the example of FIG. 2, MR system 212 includes visualization device 213. In some examples, in addition to including a visualization device, an MR system may include external computing resources that support the operations of the visualization device. For instance, the visualization device of an MR system may be communicatively coupled to a computing device (e.g., a personal computer, notebook computer, tablet computer, smartphone, etc.) that provides the external computing resources. Alternatively, adequate computing resources may be provided on or within visualization device 213 to perform necessary functions of the visualization device.

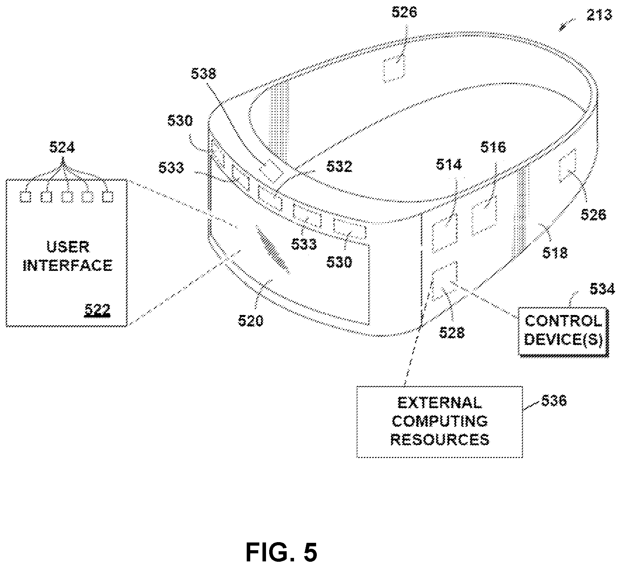

FIG. 5 is a schematic representation of visualization device 213 for use in an MR system, such as MR system 212 of FIG. 2, according to an example of this disclosure. As shown in the example of FIG. 5, visualization device 213 can include a variety of electronic components found in a computing system, including one or more processor(s) 514 (e.g., microprocessors or other types of processing units) and memory 516 that may be mounted on or within a frame 518. Furthermore, in the example of FIG. 5, visualization device 213 may include one or more display devices, such as a transparent screen 520 that is positioned at eye level when visualization device 213 is worn by a user. In some examples, screen 520 can include one or more liquid crystal displays (LCDs) or other types of display screens on which images are perceptible to a surgeon who is wearing or otherwise using visualization device 213 via screen 520. Other display examples include organic light emitting diode (OLED) displays. In some examples, visualization device 213 can operate to project 3D images onto the user's retinas using techniques known in the art.

In some examples, screen 520 may include see-through holographic lenses. sometimes referred to as waveguides, that permit a user to see real-world objects through (e.g., beyond) the lenses and also see holographic imagery projected into the lenses and onto the user's retinas by displays, such as liquid crystal on silicon (LCoS) display devices, which are sometimes referred to as light engines or projectors, operating as an example of a holographic projection system 538 within visualization device 213. In other words, visualization device 213 may include one or more see-through holographic lenses to present virtual images to a user. Hence, in some examples, visualization device 213 can operate to project 3D images onto the user's retinas via screen 520, e.g., formed by holographic lenses. In this manner, visualization device 213 may be configured to present a 3D virtual image to a user within a real-world view observed through screen 520, e.g., such that the virtual image appears to form part of the real-world environment. In some examples, visualization device 213 may be a Microsoft HOLOLENS^{™} headset, available from Microsoft Corporation, of Redmond, Washington, USA, or a similar device, such as, for example, a similar MR visualization device that includes waveguides. The HOLOLENS ^{™} device can be used to present 3D virtual objects via holographic lenses, or waveguides, while permitting a user to view actual objects in a real-world scene, i.e., in a real-world environment, through the holographic lenses.

Although the example of FIG. 5 illustrates visualization device 213 as a head-wearable device, visualization device 213 may have other forms and form factors. For instance, in some examples, visualization device 213 may be a handheld smartphone or tablet.

Visualization device 213 can also generate a user interface (UI) 522 that is visible to the user, e.g., as holographic imagery projected into see-through holographic lenses as described above. For example, UI 522 can include a variety of selectable widgets 524 that allow the user to interact with a mixed reality (MR) system, such as MR system 212 of FIG. 2. Imagery presented by visualization device 213 may include, for example, one or more 3D virtual objects. Details of an example of UI 522 are described elsewhere in this disclosure. Visualization device 213 also can include a speaker or other sensory devices 526 that may be positioned adjacent the user's ears. Sensory devices 526 can convey audible information or other perceptible information (e.g., vibrations) to assist the user of visualization device 213.

Visualization device 213 can also include a transceiver 528 to connect visualization device 213 to a processing device 510 and/or to network 208 and/or to a computing cloud, such as via a wired communication protocol or a wireless protocol, e.g., Wi-Fi, Bluetooth, etc. Visualization device 213 also includes a variety of sensors to collect sensor data, such as one or more optical camera(s) 530 (or other optical sensors) and one or more depth camera(s) 532 (or other depth sensors), mounted to, on or within frame 518. In some examples, the optical sensor(s) 530 are operable to scan the geometry of the physical environment in which user of MR system 212 is located (e.g., an operating room) and collect two-dimensional (2D) optical image data (either monochrome or color). Depth sensor(s) 532 are operable to provide 3D image data, such as by employing time of flight, stereo or other known or future-developed techniques for determining depth and thereby generating image data in three dimensions. Other sensors can include motion sensors 533 (e.g., Inertial Mass Unit (IMU) sensors, accelerometers, etc.) to assist with tracking movement.

MR system 212 processes the sensor data so that geometric, environmental, textural, etc. landmarks (e.g., corners, edges or other lines, walls, floors, objects) in the user's environment or "scene" can be defined and movements within the scene can be detected. As an example, the various types of sensor data can be combined or fused so that the user of visualization device 213 can perceive 3D images that can be positioned, or fixed and/or moved within the scene. When fixed in the scene, the user can walk around the 3D image, view the 3D image from different perspectives, and manipulate the 3D image within the scene using hand gestures, voice commands, gaze line (or direction) and/or other control inputs. As another example, the sensor data can be processed so that the user can position a 3D virtual object (e.g., a bone model) on an observed physical object in the scene (e.g., a surface, the patient's real bone, etc.) and/or orient the 3D virtual object with other virtual images displayed in the scene. As yet another example, the sensor data can be processed so that the user can position and fix a virtual representation of the surgical plan (or other widget, image or information) onto a surface, such as a wall of the operating room. Yet further, the sensor data can be used to recognize surgical instruments and the position and/or location of those instruments.

Visualization device 213 may include one or more processors 514 and memory 516, e.g., within frame 518 of the visualization device. In some examples, one or more external computing resources 536 process and store information, such as sensor data, instead of or in addition to in-frame processor(s) 514 and memory 516. In this way, data processing and storage may be performed by one or more processors 514 and memory 516 within visualization device 213 and/or some of the processing and storage requirements may be offloaded from visualization device 213. Hence, in some examples, one or more processors that control the operation of visualization device 213 may be within the visualization device, e.g., as processor(s) 514. Alternatively, in some examples, at least one of the processors that controls the operation of visualization device 213 may be external to the visualization device, e.g., as processor(s) 210. Likewise, operation of visualization device 213 may, in some examples, be controlled in part by a combination one or more processors 514 within the visualization device and one or more processors 210 external to the visualization device.

For instance, in some examples, when visualization device 213 is in the context of FIG. 2, processing of the sensor data can be performed by processing device(s) 210 in conjunction with memory or storage device(s) (M) 215. In some examples, processor(s) 514 and memory 516 mounted to frame 518 may provide sufficient computing resources to process the sensor data collected by cameras 530, 532 and motion sensors 533. In some examples, the sensor data can be processed using a Simultaneous Localization and Mapping (SLAM) algorithm, or other known or future-developed algorithm for processing and mapping 2D and 3D image data and tracking the position of visualization device 213 in the 3D scene. In some examples, image tracking may be performed using sensor processing and tracking functionality provided by the Microsoft HOLOLENS^{™} system, e.g., by one or more sensors and processors 514 within a visualization device 213 substantially conforming to the Microsoft HOLOLENS^{™} device or a similar mixed reality (MR) visualization device.

In some examples, MR system 212 can also include user-operated control device(s) 534 that allow the user to operate MR system 212, use MR system 212 in spectator mode (either as master or observer), interact with UI 522 and/or otherwise provide commands or requests to processing device(s) 210 or other systems connected to network 208. As examples, the control device(s) 234 can include a microphone, a touch pad, a control panel, a motion sensor or other types of control input devices with which the user can interact.

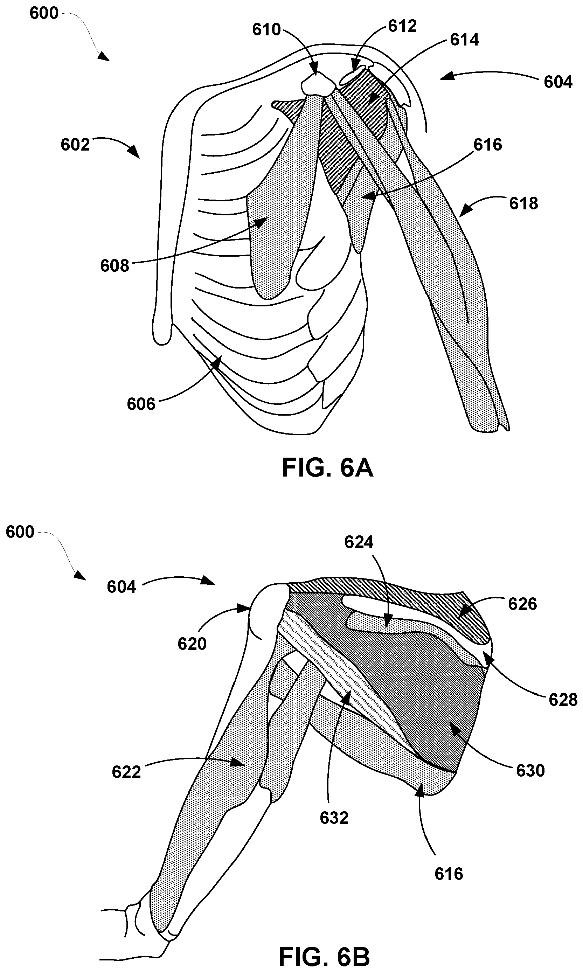

Virtual planning system 102 and/or other systems may analyze patient image data that may also be used for planning surgical intervention, such as joint surgery. As discussed herein as an example, shoulder replacement surgery is one type of surgery that may be planned using the system and techniques herein. FIGS. 6A and 6B are illustrations of example muscles and bones related to a shoulder of a patient. Using visualization device 500 in an MR system to view representations of the example muscles and bones illustrated by FIGS. 6A and 6B, surgeons may avail themselves of revision plans generated by virtual planning system 102 in performing shoulder replacement surgery.

Virtual planning system 102, implementing some of the techniques described herein, causes visualization device 213 to output representations (e.g., graphical representations) onto representations of the example muscles and bones illustrated by FIGS. 6A and 6B. Some representations indicate a specific bone quality/characteristic (e.g., by way of a corresponding color). These representations are placed in the patient-specific image data at locations in areas surrounding or underneath the removed implant. Some representations indicate locations for applying a surgical instrument, and these recommendations are placed in the patient-specific image data at locations corresponding to areas having a suitable bone quality/characteristic (e.g., "good" bone). Some representations indicate locations for implanting a replacement implant. Another representation may be an image of an actual surgical instrument at location currently being held by the surgeon's in a view of a head-mounted display device. Combined with the above representations indicating locations for applying a surgical instrument to actually remove the implant and locations for implanting a replacement implant, the patient-specific image data being presented via MR system 212 is operative to guide the surgeon though the revision surgery and removal/replacement of the implant. The other representation of the actual surgical instrument may be removed from the patient-specific image data.

As shown in the example of FIG. 6A, an anterior view of patient 600 includes sternum 602, shoulder 604, and ribs 606. Some bones associated with the structure and function of shoulder 604 include coracoid process 610 and acromion 612 of the scapula (not shown in its entirety). Muscles associated with shoulder 604 include serratus anterior 608, teres major, and biceps 618. Subscapularis 614 is one of the rotator cuff muscles shown in FIG. 6A. The other rotator cuff muscles, supraspinatus 626, infraspinatus 630, and teres minor 632 are shown in the posterior view of patient 600 in the example of FIG. 6B. FIG. 6B also illustrates the bony features of humeral head 620 and spine of scapula 628. Other muscles associated with shoulder 604 include triceps 622 and deltoid 624.

When evaluating shoulder 604 for treatment, such as what type of shoulder treatment or replacement may be appropriate, a system may analyze patient-specific image data for bones such as those discussed in FIG. 6A and 6B. For example, virtual planning system 102 may generate representations of the bone structures (e.g., bones) from the patient image data and determine bone density information including various qualities/characteristics of the bone structure. As described herein, such bone density information describes bone structures in areas occluded by an implant in a first set of patient-specific image data, and presented, after undergoing an imaging process to remove the implant, in representations of bone structures in a second set of patent-specific image data.

From this information, virtual planning system 102 may determine recommended types of treatment, such as whether or not the patient would benefit from revision surgery for an anatomical shoulder replacement or a reverse shoulder replacement. In an anatomical shoulder replacement, the humeral head is replaced with an artificial humeral head (e.g., a partial sphere), and the glenoid surface of the scapula is replaced with an artificial curved surface that mates with the artificial humeral head. In a reverse shoulder replacement, an artificial partial sphere is implanted for the glenoid surface and an artificial curved surface (e.g., a cup) that mates with the sphere is implanted in place of the humeral head. In each case, the revision surgery removes the implant and replaces it with a new implant. Virtual planning system 102 may also suggest dimensions and/or placement of replacement implants based on the patient image data, muscle characteristics, and/or bone quality/characteristics.

In one example, a system, such as virtual planning system 102, may be configured for modeling a bone structure of a patient. Virtual planning system 102 may include a memory configured to store patient-specific image data for the patient and processing circuitry. The processing circuitry may be configured to receive the patient-specific image data (e.g., CT data), determine, based on intensities of the patient-specific image data, a patient-specific shape representative of the bone structure of the patient, and output the patient-specific shape. In this manner, the patient-specific shape may be the model of the actual bone structure of the patient. As an option, the processing circuitry may be configured to receive the patient-specific image data (e.g., CT data), determine, based on intensities of the patient-specific image data, a second patient-specific shape representative of soft-tissue structure of the patient, and output the patient-specific shape. Combined into a final patient-specific shape, the patient-specific shape and the second the patient-specific shape may be the model of a shoulder of the patient.

Virtual planning system 102 may generate the patient-specific shape of the bone structure using various methods. For example, the processing circuitry may be configured to receive an initial shape (e.g., a geometric shape or statistical mean shape based on a population of patients) and determine a plurality of surface points on the initial shape. Virtual planning system 102 may then register the initial shape to the patient-specific image data and identify one or more contours in the patient-specific image data representative of a boundary of the bone structure of the patient. These one or more contours may be voxels or pixels within the patient-specific image data with intensities exceeding a threshold that indicate a boundary of the bone structure. In some examples, the contours may be determined by identifying separation zones between adjacent bone structures (e.g., using a Hessian feature image that represents intensity gradients within the patient-specific image data). A hessian feature image identifying separation zones between adjacent structures may improve the precision in which these structure boundaries as opposed to identifying the structure boundaries based on intensities alone which are very similar between bones. Virtual planning system 102 then iteratively moves the plurality of surface points towards respective locations of the one or more contours to change the initial shape to the patient-specific shape representative of the bone structure of the patient. In this manner, each iteration of the movement causes the modified initial shape to get increasingly more similar to the actual shape of the patient's bone structure as indicated in the image data.

In some examples, to generate a final patient-specific shape to include one or more representations of bone, patient-specific image data in which initial or intermediate shapes associated with a soft-tissue structure are registered to bone structures. In some examples, virtual planning system 102 may move surface points of the initial shape or intermediate shape a greater distance, or the full distance, towards a contour based on the identified intensity value of the voxel or pixel at that location. For example, high intensity voxels may indicate the presence of bone. Generally, soft-tissue structures may be disposed against a portion of bone. Therefore, if the voxel is identified to be bone, virtual planning system 102 may move the respective surface point of the initial shape or intermediate shape directly to, or adjacent to, the identified bone structure. In other examples, virtual planning system 102 may increase the tolerance of the modification distance when bone is identified as part of the contour to enable the next iteration to more precisely approximate the contour of the bone. In other examples, as discussed herein, the contour may be determined based on the Hessian feature image representing separation zones between adjacent structures. In some examples, virtual planning system 102 may track the profile behavior of the Hessian feature image along the vector in order to determine the correspondence to the border of the bone structure. The Hessian feature image may include a profile similar to a rectangle-like function that provides a voxel for correspondence for the vector. For bone structures, virtual planning system 102 may know the voxel of the bone surface in order to move the surface point directly to that voxel.

Once the final patient-specific shape is determined, virtual planning system 102 may output that patient-specific shape. In some examples, virtual planning system 102 may control the patient-specific shape to be displayed to a user. In other examples, virtual planning system 102 may perform additional calculations on patient-specific shape. For example, virtual planning system 102 may determine, a volume, linear dimensions, cross-sectional dimensions, or other characteristics of the patient-specific shape. Virtual planning system 102 may use these characteristics in other determinations as described herein.

In some examples, virtual planning system 102 may display the final patient-specific shape that has been modeled using the image data. Virtual planning system 102 may also perform additional determinations as part of the surgical plan. For example, virtual planning system 102 may use the patient-specific image data to determine a range of motion of the patient, and then determine, based on the range of motion, one type of a plurality of types of shoulder treatment procedure for the patient.

Virtual planning system 102 may determine the range of motion of the humerus by determining, based on fat volume ratios and atrophy ratios for one or more muscles of a rotator cuff of the patient, the range of motion of the humerus of the patient. Based on this information, virtual planning system 102 may select the type of shoulder treatment from one of an anatomical shoulder replacement surgery or a reverse shoulder replacement surgery. In some examples, virtual planning system 102 may recommend a reverse shoulder replacement surgery for situations when the bones and/or muscles of the patient cannot support the anatomical shoulder replacement. In this manner, patients determined to have larger fatty infiltration and larger atrophy ratios may be better suited for the reverse shoulder replacement (e.g., as compared to one or more appropriate thresholds). In some examples, planning system 102 may employ a decision tree or neural network and use the fatty infiltration values as an input along with other parameters such as patient age, gender, activity and/or other factors that may indicate whether the patient is better suited for reverse or anatomical shoulder replacement. In some examples, the fatty infiltration value may be a type of quality metric for a soft tissue structure, such as a muscle. In other examples, the quality of the muscle may be represented by another type of value that may or may not incorporate the presence of fat in the muscle.

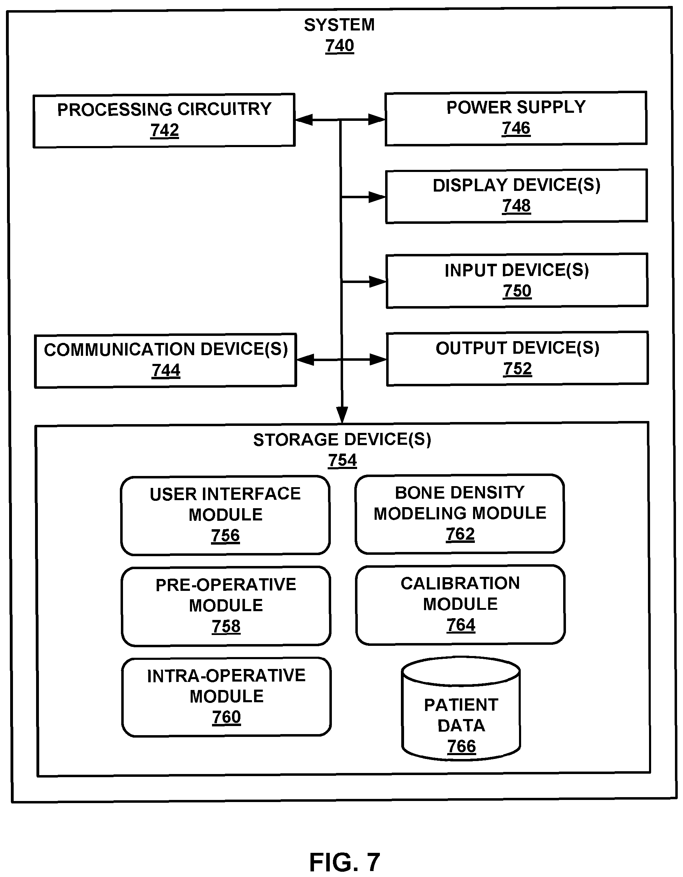

FIG. 7 is a block diagram illustrating example components of system 740 configured to determine bone structure dimensions and other information related to surgical intervention associated with a joint, according to an example of this disclosure. System 740 may be similar to virtual planning system 102 of FIG. 1 and/or systems configured to perform the processes discussed herein. In the example of FIG. 7, system 714 includes processing circuitry 742, communication devices 744, a power supply 746, display device(s) 748, input device(s) 750, output device(s) 752, and storage device(s) 754. In the example of FIG. 7, display device(s) 748 may display imagery to present a user interface to the user, such as opaque or at least partially transparent screens. Display devices 748 may present visual information and, in some examples, audio information or other information presented to a user. For example, display devices 748 may include one or more speakers, tactile devices, and the like. In other examples, output device(s) 752 may include one or more speakers and/or tactile devices. Display device(s) 748 may include an opaque screen (e.g., an LCD or LED display). Alternatively, display device(s) 748 may include an MR visualization device, e.g., including see-through holographic lenses, in combination with projectors, that permit a user to see real-world objects, in a real-world environment, through the lenses, and also see virtual 3D holographic imagery projected into the lenses and onto the user's retinas, e.g., by a holographic projection system such as the Microsoft HOLOLENS^{™} device. In this example, virtual 3D holographic objects may appear to be placed within the real-world environment. In some examples, display devices 748 include one or more display screens, such as LCD display screens, OLED display screens, and so on. The user interface may present virtual images of details of the virtual surgical plan for a particular patient.

In some examples, a user may interact with and control system 740 in a variety of ways. For example, input devices 760 may include one or more microphones, and associated speech recognition processing circuitry or software, may recognize voice commands spoken by the user and, in response, perform any of a variety of operations, such as selection, activation, or deactivation of various functions associated with surgical planning, intra-operative guidance, or the like. As another example, input devices 760 may include one or more cameras or other optical sensors that detect and interpret gestures to perform operations as described above. As a further example, input devices 760 include one or more devices that sense gaze direction and perform various operations as described elsewhere in this disclosure. In some examples, input devices 760 may receive manual input from a user, e.g., via a handheld controller including one or more buttons, a keypad, a keyboard, a touchscreen, joystick, trackball, and/or other manual input media, and perform, in response to the manual user input, various operations as described above.

Communication devices 744 may include one or more circuits or other components that facilitate data communication with other devices. For example, communication devices 744 may include one or more physical drives (e.g., DVD, Blu-ray, or universal serial bus (USB) drives) that allow for transfer of data between system 740 and the drive when physically connected to system 740. In other examples, communication devices 744 may include. Communication devices 744 may also support wired and/or wireless communication with another computing device and/or a network.

Storage devices 744 may include one or more memories and/or repositories that store respective types of data in common and/or separate devices. For example, user interface module 756 may include instructions that define how system 740 controls display devices 748 to present information to a user. Pre-operative module 758 may include instructions regarding analysis of patient data 766, such as image data, and/or determination of treatment options based on patient data 766. Intra-operative module 760 may include instructions that define how system 740 operates in providing information to a clinician for display such as details regarding the planned surgery and/or feedback regarding the surgical procedure.

Processing circuitry 742 may determine representations of bone (e.g., patient-specific shapes) from patient-specific image data. For example, processing circuitry 742 may specify initial shapes, number of iterations, and other details regarding adjusting the initial shapes to the patient-specific shapes based on the intensities of the patient image data. Processing circuitry 742 may execute instructions defining how to register the initial shape or other anatomical structures to patient-specific image data. For example, processing circuitry 742 be instructed how to register a statistical shape model (SSM) or a statistical mean shape (SMS) (e.g., an anatomical shape derived from a population of many people) with the bones of patient-specific image data prior to generating the patient-specific shape during a surface fitting process. Patient data 766 may include any type of patient data, such as patient-specific image data (e.g., CT scan, X-ray scan, or MRI data), bone density information, patient characteristics (e.g., age, height, weight), patient diagnoses, patient conditions, prior surgeries or implants, or any other information related to the patient.

Patient data 766 may include anatomy scans as examples of computed tomography (CT) scans of a patient, e.g., as represented by CT scan image data. Anatomy scans may be sufficient to construct a three-dimensional (3D) representation of the anatomy of the patient, such as the scapula and glenoid, by either automated or manual segmentation of the CT image data to yield segmented anatomical objects. One example implementation of automated segmentation is described in U.S. Patent No. 8,971,606. There may be various other ways in which to perform automated segmentation, and the techniques are not limited to automated segmentation using techniques described in U.S. Patent No. 8,971,606. As one example, segmentation of the CT image data to yield segmented objects includes comparisons of voxel intensity in the image data to determine bony anatomy and comparisons to estimated sizes of bony anatomy to determine a segmented object. Moreover, the example techniques may be performed with non-automated segmentation techniques, where a medical professional evaluates the CT image data to segment anatomical objects, or some combination of automation and user input for segmenting anatomical objects.

In one or more examples, anatomy scans may be scans of anatomy that include implants, and hence, are pathological due to injury or disease. The patient may have an injured shoulder requiring a revision procedure, and for the procedure or possibly as part of the diagnosis, the surgeon may have requested anatomy scans to plan the surgery. A computing device may generate segmentations of the patient anatomy so that the surgeon can view anatomical objects and the size, shape, and interconnection of the objects with other anatomy of the patient anatomy needing surgery.

Example ways in which to segment out a first anatomical object are described in U.S. Provisional Application Serial Nos. 62/826,119, 62/826,133, 62/826,146, 62/826,168, and 62/826,190 all filed on March 29, 2019 and U.S. Provisional Application Serial No. 62/887,838 filed on August 16, 2019. There may be other example ways in which to segment out the first anatomical object.

As one example, for segmenting, processing circuitry 742 may utilize differences in voxel intensities in image data to identify separation between bony regions and tissue regions to identify the first anatomical object. As another example, for segmenting, processing circuitry 742 may utilize closed-surface fitting (CSF) techniques in which processing circuitry 742 uses a shape model (e.g., predetermined shape like a sphere or a shape based on statistical shape modeling) and expands or constricts the shape model to fit a contour used to identify separation locations between bony regions and tissue or between tissue.

As discussed above, surgical lifecycle 300 may include a preoperative phase 302 (FIG. 3). One or more users may use orthopedic surgical system 100 in preoperative phase 302. For instance, orthopedic surgical system 100 may include virtual planning system 102 (with may be similar to system 740) to help the one or more users generate a virtual surgical plan that may be customized to an anatomy of interest of a particular patient. As described herein, the virtual surgical plan may include a 3-dimensional virtual model that corresponds to the anatomy of interest of the particular patient and a 3-dimensional model of one or more prosthetic components matched to the particular patient to repair the anatomy of interest or selected to repair the anatomy of interest. The virtual surgical plan also may include a 3-dimensional virtual model of guidance information to guide a surgeon in performing the surgical procedure, e.g., in preparing bone surfaces or tissue and placing implantable prosthetic hardware relative to such bone surfaces or tissue.

As discussed herein, system 740 may be configured to model a bone structure of a patient using patient image data. For example, system 740 may include a memory (e.g., storage devices 754) configured to store patient-specific image data for the patient (e.g., patient data 766). System 740 also includes processing circuitry 742 configured to receive the patient-specific image data and determine, based on intensities of the patient-specific image data, a patient-specific shape representative of the bone structure of the patient. Processing circuity 742 can then output the patient-specific shape, such as for display or use in further analysis for the patient. For example, processing circuitry 742 may use the patient-specific shape or other characteristics from the patient-specific image data to generate surgical procedure recommendations (e.g., which type of treatment should be performed on a patient) as described herein.

Processing circuitry 742 may determine the patient-specific shape using one or more processes, such as a surface fitting process. For example, processing circuitry 742 may receive an initial shape (e.g., a geometric shape, a statistical mean shape (SMS), or a statistical shape model (SSM)), determine a plurality of surface points on the initial shape, and register the initial shape to the patient-specific image data. Processing circuitry 742 may register the initial shape by determining one or more pre-segmented bones in the patient-specific image data or otherwise identifying an approximate location of bone structure(s) of interest. In other examples, processing circuitry 742 may register the initial shape as soft-tissue structure of interest or a bone structure, for instance, by determining that no soft tissue structure of interest fits the initial shape. Processing circuitry 742 may then identify one or more contours in the patient-specific image data representative of a boundary of the bone structure (which may be based on a separation zone between bone structures, such as between a scapula and a humerus) of the patient and iteratively move the plurality of surface points towards respective locations of the one or more contours to change the initial shape to the patient-specific shape representative of the bone structure of the patient. In this manner, processing circuitry 742 may generate one or more intermediate shapes as the boundary of the initial shape is iteratively moved towards a closer fit to the contours. The contours may represent a collection of voxels that exceed a certain threshold, or fall within a threshold range, indicative of a boundary of the bone structure. As an option, processing circuitry 742 may identify a portion of the initial shape as soft-tissue structure by determining one or more muscle insertion points and/or origins on pre-segmented bones in the patient-specific image data or otherwise identifying an approximate location of soft tissue structure(s) of interest.

In some examples, to generate a final representation of bone structure(s), processing circuitry 742 may move surface points of the initial shape or intermediate shape a greater distance, or the full distance, towards a contour based on the identified intensity value of the voxel or pixel at that location. For example, high intensity voxels may indicate the presence of bone. Generally, soft-tissue structures may be disposed against a portion of bone. Therefore, if the voxel is identified to be bone, processing circuitry 742 may move the respective surface point of the initial shape or intermediate shape directly to, or adjacent to, the identified bone structure. In other examples, processing circuitry 742 may increase the tolerance of the modification distance when bone is identified as part of the contour to enable the next iteration to more precisely approximate the contour of the bone. In other examples, the contour may be determined based on the Hessian feature image representing separation zones between adjacent structures. In some examples, processing circuitry 742 may track the profile behavior of the Hessian feature image along the vector in order to determine the correspondence to the border of the bone structure. The Hessian feature image may include a profile similar to a rectangle-like function that provides a voxel for correspondence for the vector. For bone structures, processing circuitry 742 may know the voxel of the bone surface in order to move the surface point directly to that voxel.

In some examples, the initial shape and the patient-specific shape are three-dimensional shapes. However, in other examples, the initial shape and/or the patient-specific shape may be defined in two dimensions. A set of several two-dimensional shapes may be used to define an entire volume, or three-dimensional shape, in these examples. In one example, processing circuitry 742 may iteratively move the surface points of the initial shape, and intermediate shapes, in the direction of respective vectors in three dimensions such that processing circuitry 742 processes data in a three-dimensional space. In other examples, processing circuitry 742 may operate in two-dimensional slices to change the initial shape towards the contours in the patient-specific image data. Then, processing circuitry 742 may combine the several two-dimensional slices to generate the full three-dimensional volume of the final patient-specific shape for the patient.

A bone structure refers to any one of 216 bones in a human body including external and internal structures thereof. Revision surgeries for joint replacement treatments generally involve modification of a bone (e.g., replacing a pre-existing implant and (possibly) a portion of the bone with artificial materials such as metal and/or polymers). In this manner, system 740 may analyze bone structure of the patient, such as the bones that form a joint, for information that may influence a surgical plan and/or a type of implant replacement. In the case of a revision surgery for a shoulder replacement, the bone structures of interest for the joint may include the scapula (e.g., glenoid shape or glenoid volt (e.g., bone stock)) and the humerus (e.g., diaphysis and metaphysis). For the purposes of surgical planning, system 740 may determine various characteristics of each bone structure for the purposes of determining to what types of range of motion and/or stresses to which the new repaired joint may be subjected.

A soft tissue structure may include a muscle, tendon, ligament, or other connective tissue that is not bone. Even though revision surgeries for joint replacement treatments generally involve modification of the bone (e.g., replacing at least a portion of the bone with artificial materials such as metal and/or polymers), soft tissue states may inform what types of replacements may be appropriate for the particular implant being replaced. In this manner, system 740 may analyze the soft tissue of the patient, such as the muscles around the joint, for information that may influence the type of implant replacement. In the case of a shoulder replacement, the soft tissue structures of interest for the joint may include the rotator cuff muscles, such as the subscapularis, supraspinatus, infraspinatus, and teres minor. Other muscles associated with the shoulder, such as the teres major, deltoid, serratus anterior, triceps, and biceps, may be analyzed for revision surgery for shoulder replacement treatment as well. For the purposes of surgical planning, system 740 may determine various characteristics of each soft tissue structure for the purposes of determining to what types of range of motion and/or stresses to which the new repaired joint may be subjected.

In some examples, processing circuitry 742 may determine a type of implant replacement for the patient based on various criteria, such as the range of motion of the humerus with respect to the glenoid surface or rest of the scapula. The implant replacement may be a different type of shoulder treatment that the one being revised. Types of shoulder treatment may include an anatomical shoulder replacement or a reverse shoulder replacement, and processing circuitry 742 may suggest which type of replacement is preferred for the patient based on the bone qualities/characteristics. In addition, processing circuity 742 recommend other parameters for the treatment, such as implant placement locations, angles, orientations, type of implant, etc. For example, processing circuitry 742 may determine areas having "good" bone and areas having "bad" bone for the patient-specific shape from the patient-specific image data. From this information, processing circuitry 742 may suggest a type of shoulder treatment for the patient during the preoperative planning phase.

Bone density modeling module 762 may include instructions defining how processing circuitry 742 determines bone density information (e.g., bone density measurements indicating bone qualities/characteristics) for at least a portion of one or more bones, such as the humeral head. For example, bone density modeling module 762 may determine bone density information based on intensity of voxels within patient data 766 (e.g., CT image data). Processing circuitry 742 may execute bone density modeling module 762 to determine different bone qualities/characteristics of groups of pixels or voxels according to predetermined ranges of intensity (e.g., Hounsfield units) for individual or groups of pixels or voxels. In some examples, processing circuitry 742 may generate the bone density measurements based on the standard deviation of voxels within the patient-specific image data. The bone density measurements may include different bone density values across a two-dimensional or three-dimensional region of the humeral head. In some examples, the bone density measurement may be a single value determined based on the average pixel or voxel intensities across the humeral head or in a certain area of the humeral head, and depending on the range of pixel or voxel intensities being determined by bone density modeling module 762, that single value may indicate a specific bone quality/characteristic (e.g., "good" bone).

In some examples, bone density modeling module 762 may include instructions that determine which type of replacement humeral implant (e.g., stemmed or stemless) to implant and/or the location (e.g., two-dimensional or three-dimensional area) at which the humeral implant can be implanted within the humeral head. The bone density information may not actually indicate the density of bone but may be a measurement representative of bone density and in accordance with a bone density metric defining bone densities. For example, the bone density information may include numerical values and based upon these values, bone density modeling module 762 determines the type of implant (e.g., stemmed or stemless) to recommend as the replacement implant for the implant currently implanted into the patient and/or the type of implant (e.g., stemmed or stemless) currently implanted into the patient, as indicated by patient-specific image data.

In some examples, bone density modeling module 762 analyzes bone density historical data associated with a plurality of revision surgeries to build and train a statistical model to predict which replacement implant should be used. The bone density information may include values that correlate with a certain type of replacement implant (e.g., stemmed or stemless) to recommend, for instance, in medical terms such as curative value, remedial effects, likelihood of success, pain, and overall medical benefit that corresponds to the analyzed patient-specific image data. As another example, the bone density information may include voxel intensity from image data, standard deviations of voxel intensity from image data, compressibility, an index, or some other indication that may be related to, or representative of, density without actually providing a measure of the density of the bone.

Processing circuitry 742 may execute calibration module 764 to calibrate the bone density metric to patient-specific image data and selected implant types from other patients in historical surgeries (e.g., implant types historically selected based on thumb test information during that surgery). Historically, a clinician may use their thumb to press against the trabecular bone within the humeral head (exposed by the cut head) to determine the stiffness, and thus density, of the trabecular bone. This thumb test may be performed in order to identify what type of stem, if any, is needed for the humeral implant. Calibration module 764 may use this thumb test data obtained from historical patients to correlate known surgical decisions of humeral implant type made based on thumb test procedures to patient-specific image data of the same respective patient to determine bone density information for the current patient. In this manner, calibration module 764 may be used to identify one or more ranges of bone density information that correspond to respective humeral implant types. For instance, with calibration module 764, processing circuitry 742 may determine that stemless humeral implant is for bone density metrics within a first range, short stemmed humeral head is for bone density information within a second range, and long-stemmed humeral head is for bone density information within a third range.

As discussed above, surgical lifecycle 300 may include a preoperative phase 302 (FIG. 3). One or more users may use orthopedic surgical system 100 in preoperative phase 302. For instance, orthopedic surgical system 100 may include virtual planning system 102 (with may be similar to system 740) to help the one or more users generate a virtual surgical plan that may be customized to an anatomy of interest of a particular patient. As described herein, the virtual surgical plan may include a 3-dimensional virtual model that corresponds to the anatomy of interest of the particular patient and a 3-dimensional model of one or more prosthetic components (e.g., implants) matched to the particular patient to repair the anatomy of interest or selected to repair the anatomy of interest. The virtual surgical plan also may include a 3-dimensional virtual model of guidance information to guide a surgeon in performing the surgical procedure, e.g., in preparing bone surfaces or tissue and placing implantable prosthetic hardware relative to such bone surfaces or tissue.

As discussed herein, bone density modeling module 762, executing on processing circuitry 742, may be configured to determine bone density information for at least a portion of a humeral head of a patient based on the patient-specific image data for that patient. For example, the bone density information may include a bone density measurement as a single indication of overall density of the humeral head or a portion of the humeral head. As another example, the bone density information may include bone density values for respective portions of a humeral head of the patient. The system 740 may control a user interface via user interface module 756 to present a graphical representation of the bone density information (which may be directly or indirectly indicative of bone density) and/or generate a recommendation on the implant type for the humeral head based on the bone density metric. For example, a bone density measurement indicative of sufficient trabecular bone density in the humeral head may result in the system recommending a stemless humeral implant as opposed to a stemmed humeral implant.

In one example, processing circuitry 742 may be configured to identify a humeral head in the patient-specific image data, such as by segmenting the bone or otherwise identifying landmarks or shapes indicative of the humeral head. Processing circuitry 742 may then determine, based on the patient-specific image data, bone density information representing bone density of at least a portion of the humeral head. Based on this bone density information, processing circuitry 742 executing bone density modeling module 762 may generate a recommendation of a humeral implant type for the patient undergoing revision surgery for a previously installed implant. For example, processing circuitry 742 may recommend a stemmed humeral implant (stemmed implant type) for bone density metrics indicative of less dense bone and processing circuitry 742 may recommend a stemless humeral implant (stemless implant type) for bone density information indicative of higher density bone. Processing circuitry 742 may then output, for display via a user interface, the recommendation of the replacement humeral implant type for the patient.

In some examples, processing circuitry 742 may determine a stem length for a humeral implant type that includes a stem. Processing circuitry 742 may determine that less dense bone requires longer stems to provide sufficient anchoring to the humerus or determine that the locations of lower density trabecular bone within the humerus requires a longer stem. The stem length itself may be identified and presented to the user, or processing circuitry 742 may recommend certain humeral implants satisfying the recommended length range. In this manner, processing circuitry 742 may recommend a specific replacement implant or implant type selected between three or more different types of humeral implants based on the bone density information determined from the patient-specific image data.

In some examples, the bone density metric may represent an overall density score (e.g., a value, index, or category based on voxel or pixel values from image data) for trabecular bone within at least a portion of the humeral head. For example, processing circuitry 742 may determine an averaged or weighted average density for a region of the humeral head and assign a specific metric value to that region of the humeral head. In other examples, the bone density metric may be determined to be indicative of the lowest density of bone found in the region to establish a lower limit on the bone density in that area. Conversely, the bone density metric may be indictive of the highest density in that region of the humeral head. The bone density metric may include a plurality of bone density values for respective portions within the humeral head. For example, the bone density metric may include a matrix of density values that includes specific bone density values for respective voxels, or groups of voxels, within a region of the humeral head. In this manner, the bone density metric may provide a higher resolution representation of the bone density within the humeral head. In any case, the bone density metric may be indicative of actual bone density values, image data intensities, and/or recommended implant types).

Processing circuitry 742 may determine the bone density information using different metrics. In one examples, processing circuitry 742 may define, for a bone density metric, two or more intensity levels corresponding to bone qualities/characteristics and determine a measurement from the bone density metric by identifying, based on the patient-specific image data, intensities of respective voxels within at least a portion of the humeral head, classifying the intensities of the respective voxels in one of two or more intensity levels, and determining, based on at least one of a number of voxels classified within each of the two or more intensity levels or a location in the humeral head of the voxels classified within each of the two or more intensity levels, the bone density measurement. In this manner, processing circuitry 742 may be configured to classify different intensities in the patient-specific image data as different intensity levels and/or the location of those intensity levels to determine the bone density measurement. For example, the location of the intensity levels may be relevant to whether or not the trabecular bone is dense enough to support a stemless humeral implant. If the trabecular bone has a lower overall bone density, but the center of the humeral head is still above a threshold density required to support a stemless humeral implant, processing circuitry 742 may still determine that the bone density measurement is sufficient to support a stemless humeral implant. In other examples, processing circuitry 742 may determine the bone density measurement as indicative of requiring a stemmed humeral implant even with some relatively high bone density levels if pockets of low-density trabecular bone are identified in locations at which a stemless humeral implant would be implanted.

In some examples, processing circuitry 742 may determine the bone density measurement for a volume of trabecular bone within the entire humeral head. In other examples, processing circuitry 742 may determine a plane through a humeral head representative of a humeral cut made in the humerus to prepare the humerus for accepting a humeral implant. This humeral cut would expose the surface of the trabecular bone in which the humeral implant would be implanted. The processing circuitry 742 would then determine the bone density measurement for at least a portion of the humeral head bisected by the plane. In some examples, processing circuitry 742 may determine the bone density measurement for pixels or voxels that correspond to the plane (e.g., are exposed by or bisected by the plane). In other examples, processing circuitry 742 may determine the bone density measurement for a volume of trabecular bone starting at the plane and extending towards the shaft of the humerus. In some examples, the volume of analyzed trabecular bone may extend up to cortical bone that defines the outer surface of the humerus.

The bone density information may be displayed via a user interface, such as using user interface module 1156, in some examples. Processing circuitry 742 may output, for display by display devices 1148 or a display device of another system, the user interface comprising a graphical representation of the bone density information over a representation of at least a portion of the humeral head of the patient. The graphical representation of the bone density information may include a two or three dimensional graphic that may include one or more shapes or colors that is displayed over or in place of the trabecular bone of the humerus. In one example, the bone density information may include a heat map of a plurality of colors, where each color of the plurality of colors represents a different range of bone density values. In this manner, different colors may represent different bone density magnitudes to indicate a spatial representation of the variation in bone density within that volume of trabecular bone. The graphical representation of the bone density information may include a two-dimensional representation of bone density variation within a plane of the humeral head. In other examples, the graphical representation of the bone density information may include a three-dimensional representation of bone density variation within at last trabecular bone of the humeral head. In some examples, display devices 1148 may include a mixed reality display, and processing circuitry 742 may control the mixed reality display to present the user interface comprising the graphical representation of the bone density information.

In some examples, the bone density information includes measurements that are correlated with bone density data (e.g., image data or other data indicative of bone structure in the humeral head) from other historical patients and the type of humeral implant selected by the clinician for that particular bone density data. The bone density data may be generated for these historical patients using the patient-specific image data for each patient and the resulting type of humeral implant selected by the surgeon for each respective patient (e.g., which may be based on a "thumb test" where the clinician uses their thumb to press against the trabecular bone in the humeral head and classifies the trabecular bone as sufficient or insufficient for a stemless humeral implant). Processing circuitry 742 may leverage these selected implant types based on the thumb test to classify bone density measurements as suitable or not suitable for stemless humeral implants in future patients. In this manner, processing circuitry 742 may correlate the bone density measurements with type of replacement humeral implant selected by surgeons in previously performed surgeries on other subjects, where the thumb test data is indicative of manually determined density ranges (or compressibility which is representative of bone density) of trabecular bone within respective humeral heads of the other subjects. Based on this correlation, processing circuitry 742 may determine the recommendation of the humeral implant type for the patient. In some examples, processing circuitry 742 may employ one or more neural networks to correlate the previous selected implant type and respective patient-specific image data to determine a bone density measurement or set of measurements indicative of each type of implant available for future patients. For example, processing circuitry 742 may use the bone density measurements, patient-specific image data, and selected humeral implant type (stemmed, stemless, and/or length of stem) as inputs to the neural network. The outputs of the neural network may be those bone density measurements that correspond to which humeral implant type.

In some examples, processing circuitry 742 may generate a shoulder surgery recommendation for a patient using bone qualities/characteristics and (possibly) soft tissue characteristics. For example, processing circuitry 742 may determine, based on the patient-specific image data, one or more soft tissue characteristics (e.g., soft tissue volume, fatty infiltration ratio, atrophy ratio and/or range of motion value) in addition to bone density information associated with a humerus of the patient. As described herein, processing circuitry 742 may generate a recommendation of a shoulder surgery type to be performed for the patient (e.g., an anatomical or reverse shoulder surgery) and generate, based on the bone density measurement(s) associated with the humerus, a recommendation of a humeral implant type for the patient. Processing circuitry 742 may then output, for display, the recommendation of the shoulder surgery type and the humeral implant type for the patient. In some examples, the user interface may include the representation of the one or more bone qualities/characteristics associated with the humerus as part of a mixed reality user interface.

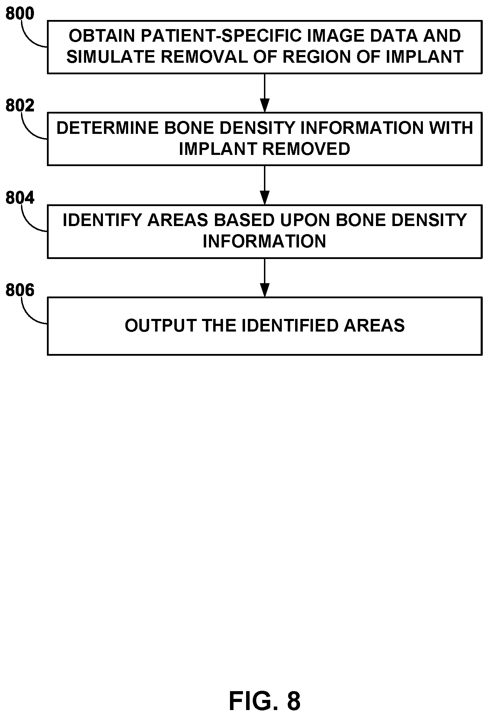

FIG. 8 is a flowchart illustrating an example procedure for modeling bone density information for a revision surgery, in accordance with a technique of this disclosure. Processing circuitry 742 of system 740 will be described as performing the example of FIG. 8, but other devices or systems, such as virtual planning system 102, may perform one or more portions of this technique. Furthermore, some portions of this technique may be performed by a combination of two or more devices and/or systems via a distributed system. The process of FIG. 8 is described with respect to three-dimensional data sets, but several two-dimension slices of data may be analyzed in a similar manner in other examples.

As shown in FIG. 8, processing circuitry 742 may obtain patient-specific image data and simulate removal of a region of an implant on the patient's bone (800). Being of medical interest, the patient-specific image data may be generated by a healthcare facility using or more imaging modalities (e.g., x-ray, CT, MRI, etc.) and stored in a secured data storage device. As described herein, processing circuitry 742 applies an imaging process that segments the region of the implant and then, removes the segmented region from the patient-specific image data, (in effect) generating a second set of patient-specific image data.

When obtaining image data and segmenting the image data, the presence of an existing implant, particularly a metallic implant, can potentially cause complications with the segmentation process. There are a number of techniques to successfully overcome these complications in an effective segmentation process, such as the ones described in Provisional Application Serial No. 62/887 838. The techniques use artificial suppression to overcome or avoid the complications created by the noise and artifacts caused by implants and enable a computing device to produce a useful segmentation of a joint, even when the joint includes an implant component. Furthermore, some techniques that may enable a device to obtain, from acquired image data, information regarding the one or more existing implant components and the joint. At least one example technique described herein may segment the image data to separate out an existing implant, particularly a metallic implant, reforming a joint (e.g., a shoulder joint formed by the scapula and the humerus).

Processing circuitry 742 determines bone density information with the implant removed (802). As described herein, processing circuitry 742 accesses intensity values for the segmented region of the implant and determines bone density measurements for areas of bone around the segmented region. In some examples, Hounsfield units, which are values approximating bone density measurements, are computed for the segmented region of the removed implant. As one example, in the image data with data for the implant removed, pixels are associated with a relative radiodensity value corresponding to a mean attenuation, as measured in Hounsfield units (HUs) using the Hounsfield scale.

For example, processing circuitry 742 may obtain a set of CT images of a bone, such as a humerus or a scapula. Each of the CT images of the bone corresponds to a 2-dimensional slice of the bone after removal of the implant from the segmented region. Furthermore, for each of the CT images of the bone, processing circuitry 742 may partition the CT image into a set of regions and determine a map of Hounsfield unit values for the regions. In general, higher Hounsfield unit values correspond with greater bone density. Hence, cortical bone (e.g., "good" bone) may have higher Hounsfield unit values than cancellous bone (e.g., "bad" bone). For pixels/voxels of regions other than the segmented region of the removed implant, processing circuitry 742 applies the Hounsfield scale to compute the Hounsfield unit values. For pixels/voxels of the segmented region of the removed implant, processing circuitry 742 applies any one of a number of techniques for computing the Hounsfield unit values. One example technique determines the Hounsfield unit values based upon Hounsfield unit values of bone areas around the segmented region.

Processing circuitry 742 may determine a 3D model of at least a relevant part of the bone by layering the maps of Hounsfield unit values. Thus, there may be a Hounsfield unit value for each voxel (3-dimensional position) in the 3D model. Processing circuitry 742 may then use the 3D model to determine bone quality values for locations on a surface of the bone. For instance, in an example where the bone quality value for a location on the surface of the bone corresponds to a bone quality of the bone along a potential insertion axis orthogonal to the surface of the bone at the location, processing circuitry 742 may determine the bone quality value for the location based on Hounsfield unit values of voxels intersected by the potential insertion axis. For instance, processing circuitry 742 may determine the bone quality value for the location as a sum of Hounsfield unit values of the voxels intersected by the potential insertion axis. In another instance, processing circuitry 742 may determine the bone quality value for the location as a sum of Hounsfield unit values of values intersected by the potential insertion axis that are above a specific threshold (e.g., so as to exclude voxels corresponding to cancellous bone). The specific threshold may be a Hounsfield unit value indicating sufficiency of bone density in a bone area.

Processing circuitry 742 identifies areas of the region of the removed implant based upon bone density information (804). These areas are two-dimensional areas, configurable in size, and proximate to the removed implant. As described herein, processing circuitry 742 uses the bone density information to distinguish areas having sufficient bone density from areas having insufficient bone density. In some examples, processing circuitry 742 captures an area's Hounsfield unit(s) for comparison with a threshold; if that area's Hounsfield unit(s) is/are greater, processing circuitry 742 classifies the area of the region as sufficient in bone density indicating healthy bone quality/characteristics but if Hounsfield unit(s) is/are below another threshold or the same threshold, processing circuitry 742 classifies the area of the region as insufficient and indicative of unhealthy bone quality/characteristics.

Processing circuitry 742 outputs the identified areas (806). As described herein, processing circuitry 742 generates one or more representations of the bone densities existing on the identified areas. Based upon Hounsfield unit(s) of a bone area proximate to the removed implant, processing circuitry 742 may generate a representation indicating a bone quality/characteristic. Color may be used for the representation such that a specific color indicates a classification of the bone area of the region as cancellous or insufficient in bone density. Another color may be used to indicate that a bone area of the region is cortical or sufficient in bone density.

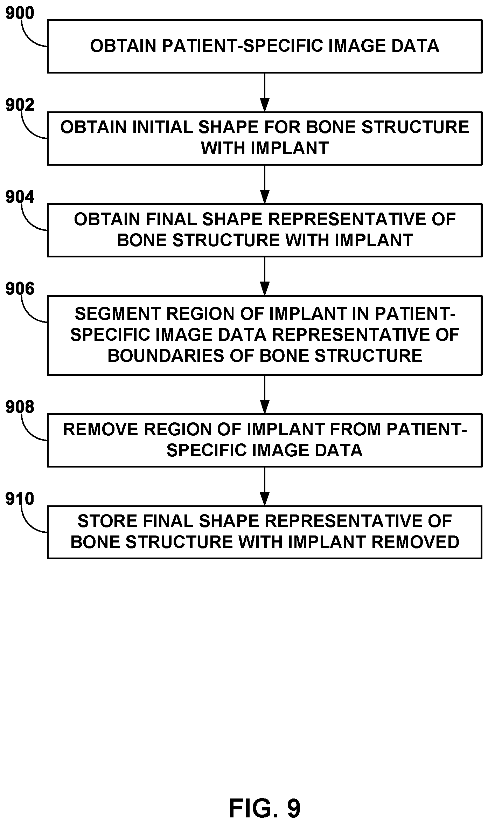

FIG. 9 is a flowchart illustrating an example procedure for modeling bone structure with an implant removed using patient-specific image data, in accordance with a technique of this disclosure. Processing circuitry 742 of system 740 will be described as performing the example of FIG. 9, but other devices or systems, such as virtual planning system 102, may perform one or more portions of this technique. Furthermore, some portions of this technique may be performed by a combination of two or more devices and/or systems via a distributed system. The process of FIG. 9 is described with respect to three-dimensional data sets, but several two-dimension slices of data may be analyzed in a similar manner in other examples.

As shown in FIG. 9, processing circuitry 742 may obtain patient-specific image data of the patient's implant and bone of interest (900). This patient-specific image data may be generated from one or more imaging modalities (e.g., x-ray, CT, MRI, etc.) and stored in a data storage device. Processing circuitry 742 then obtains an initial shape for the bone structure of interest (902). As described herein, the bone structure of interest is a bone structure with an implant in a region. The initial shape may be a geometric shape or a statistical mean shape (SMS). This bone structure may be a bone or other non-soft tissue structure, such as a humerus or scapula forming a shoulder joint. Processing circuitry 742 then registers the initial shape to the patient-specific image data. This registration may include registering the initial shape to bones and/or bone insertion points identified by unsegmented bones or already segmented bones in the patient-specific image data. In other examples where a preliminary bone segmentation has already been performed on the bone structure of interest in the patient-specific image data, processing circuitry 742 may register the initial shape to the preliminary bone segmentation. This registration may further include registering the initial shape to soft tissue structures that surround the bone structure of interest.

Processing circuitry 742 then obtains a final shape for the bone structure of interest which is a bone structure with a region of an implant (904). Processing circuitry 742 identifies one or more contours in the patient-specific image data representative of boundaries of the bone structure. These one or more contours may be identified as voxels associated with unsegmented bones or pre-segmented bones and/or a muscle in the patient-specific image data. In other examples, processing circuitry 742 may determine each contour by extending normal vectors from the surface of the initial shape inwards and/or outwards from the initial shape. Voxels or pixels encountered by each vector that exceed a threshold intensity value in the patient-specific image data may be identified as defining at least part of the contour.

Processing circuitry 742 then moves surface points on the surface of the initial shape towards respective points on the one or more contours. Movement of these surface points causes the entire surface of the initial shape to be deformed. If processing circuitry 742 determines that the surface points need to be moved again in order to more closely fit the initial shape to the one or more contours, processing circuitry 742 again moves the surface points of the deformed surface of the initial shape. When processing circuitry 742 determines that the surface points do not need to be moved again and the deformed shape fits the one or more contours, processing circuitry 742 stores and (possibly) outputs the final deformed shape as a patient-specific shape representative of the bone structure of the patient. The patient-specific shape may be presented via a user interface and/or used for further analysis, such as part of pre-operative planning of treatment for the patient.

Processing circuitry 742 then segments a region of the implant in patent-specific image data representative of boundaries of the bone structure of interest and the implant (906). Segmentation is a technique described herein where the region of the implant is partitioned into a number of partitions containing the entirety of the implant. Some segmentation techniques use artificial suppression to overcome or avoid the complications created by the noise and artifacts from implants, enabling processing circuitry 742 to produce a useful segmentation of the region of the implant adjacent to the bone structure of interest. By placing on the region a cut plane above which the implant is removed, processing circuitry 742 identifies exactly which pixels or voxels of image data to replace in the patient-specific image data to substantially remove only the implant from the patient-specific image data. Processing circuitry 742 then removes at least a portion of the region of the implant from the patent-specific image data (908). Processing circuitry 742 may modify the image data in the pixels or voxels data for the region of removed implant to indicate intensity values typically associated with bone structures. In some examples, processing circuitry 742 may compute Hounsfield units to determine suitable intensity values for at least one of the partitions containing the implant. Processing circuitry 742 then stores, in a second set of patient-specific image data, the final shape representative of bone structure with implant removed from the patent-specific image data (910).

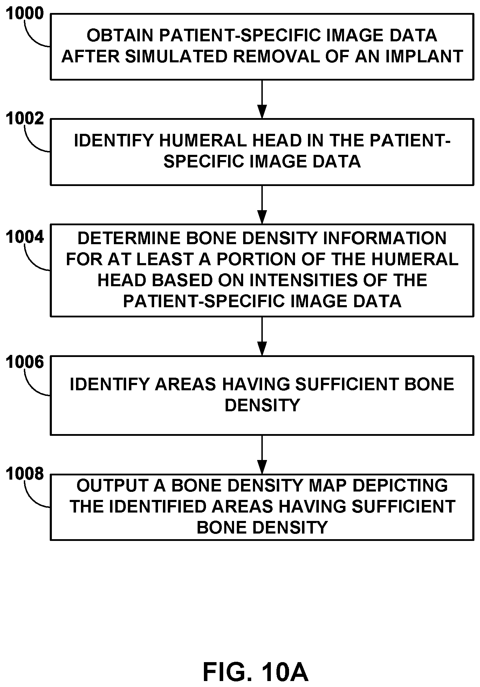

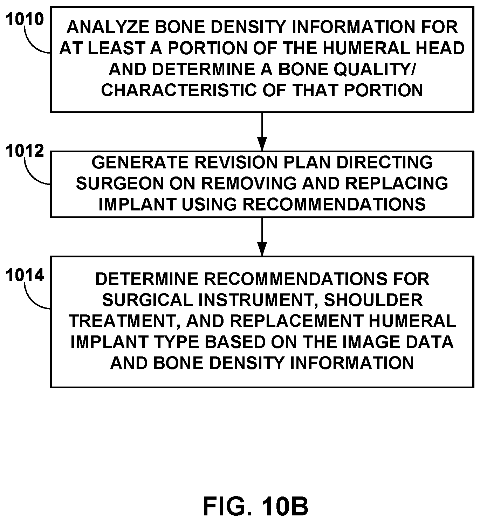

FIG. 10A is a flowchart illustrating an example procedure for presenting a representation of a bone structure model with an implant removed, in accordance with a technique of this disclosure. Processing circuitry 742 of system 740 will be described as performing the example of FIG. 10A, but other devices or systems, such as virtual planning system 102, may perform one or more portions of this technique. Furthermore, some portions of this technique may be performed by a combination of two or more devices and/or systems via a distributed system. The process of FIG. 10 is described with respect to three-dimensional data sets, but several two-dimension slices of data may be analyzed in a similar manner in other examples.

As shown in the example of FIG. 10, processing circuitry 742 may obtain patient-specific image data (e.g., from a memory or other system), such as three-dimensional CT image data, after simulated removal (1000). As described herein, the patient-specific image data being obtained includes the bone structure model of the joint and has undergone an imaging process to remove data (e.g., pixel data) for rendering a graphical representation of an implant. Processing circuitry 742 may then identify the humeral head in the patient-specific image data (1002). For example, processing circuitry 742 may segment the bones in order to identify the humeral head or determine landmarks or shapes indicative of the humeral head.

Using the patient-specific image data of the humeral head, processing circuitry 742 may determine bone density information for at least a portion of the humeral head based (in part) on intensities of the voxels or groups of voxels in the patient-specific image data (1004). By applying at least one standard metric to the patient-specific image data, the bone density information may include at least one measurement, such as a measurement indicative of an overall density of the trabecular bone within the humeral head, or a configuration of fine-grained values representing density for each voxel of groups of voxels within a region of the humeral head.

Processing circuitry 742 may then identify areas having sufficient bone density (1006). As described herein, an area's bone density can be measured in a number of ways and each metric is associated with one or more thresholds for classifying that area based upon the area's bone density. If processing circuitry 742 determines that a bone density measurement for the area is greater than a threshold, processing circuitry 742 identifies the area as having sufficient bone density. Any area having a bone density measurement below the threshold may not have sufficient bone density.

Processing circuitry 742 may then output a bone density map depicting the identified areas having sufficient bone density (1008). These areas include areas surrounding the implant prior to segmentation and simulated removal as well as areas covered or occluded by the implant such as areas underneath the implant which are exposed after segmentation and simulated removal of the implant. Having bone density information from such areas provides substantial value to revision surgery planning. Because the bone density information corresponds to exposed areas of bone, the techniques described herein provide bone density information that is not available in conventional planning systems, thereby improving an overall bone density map with respect to accuracy at the very least. In some examples, the bone density information provides sufficient bone density details to generate a highresolution bone density map, enabling pinpoint precision when directing surgical instruments to certain areas.

FIG. 10B is a flowchart illustrating an example procedure for determining recommendations for planning revision surgery based on bone density. Processing circuitry 742 of system 740 will be described as performing the example of FIG. 10B, but other devices or systems, such as virtual planning system 102, may perform one or more portions of this technique. Furthermore, some portions of this technique may be performed by a combination of two or more devices and/or systems via a distributed system. The process of FIG. 10B is described with respect to three-dimensional data sets, but several two-dimension slices of data may be analyzed in a similar manner in other examples.

As shown in the example of FIG. 10B, processing circuitry 742 may analyze bone density information obtained from patient-specific image data, such as three-dimensional CT image data, for at least a portion of the humeral head and determine a bone quality/characteristic of that portion (1010). As described herein, the patient-specific image data being obtained has undergone an imaging process to remove data (e.g., pixel data) for rendering a graphical representation of an implant. Processing circuitry 742 may then identify the humeral head in the patient-specific image data. For example, processing circuitry 742 may segment the bones in order to identify the humeral head or determine landmarks or shapes indicative of the humeral head.

Using the patient-specific image data of the humeral head, processing circuitry 742 may determine bone density information for at least a portion of the humeral head based (in part) on intensities of the voxels or groups of voxels in the patient-specific image data. By applying at least one standard metric to the patient-specific image data, the bone density information may include at least one measurement, such as an overall measurement indicative of an overall density of the trabecular bone within the humeral head, or a configuration of fine-grained values representing density for each voxel of groups of voxels within a region of the humeral head. Based upon such bone density information, processing circuitry 742 determines a bone quality or characteristic of that portion of the humeral head. As described herein, example bone qualities or characteristics include dichotomies of "good" bone/"bad" bone quality, sufficient/insufficient bone density, healthy/impaired or diseased, and/or the like. Other example bone qualities or characteristics include spectrums or ranges of classifications, such as different levels of morbidity.

Processing circuitry 742 may then generate a revision plan directing a surgeon on removing and replacing the implant (1012). In some examples, the revision plan includes, in some of the identified areas of FIG. 10A, first locations to which surgical instruments are to be applied when removing a currently implanted implant. In other examples, the revision plan includes, in some of the above-mentioned identified areas, second locations to which a replacement implant is to be implanted. Processing circuitry 742 may identify the first locations and the second locations based upon bone qualities/characteristics of the humeral head. For instance, the first locations and the second locations may identify to the surgeon areas of "good" bone such that surgeon primarily applies surgical instruments to these areas, avoiding areas of "bad" bone.

Processing circuitry 742 may then determine one or more recommendations for the revision surgery (1014). Such a recommendation may be directed to improving the planning and/or performance of the revision surgery, such as a recommendation for surgical instrument, shoulder treatment, and replacement humeral implant type based on the image data and bone density information. Processing circuitry 742 may then output the one or more recommendations to a display device and/or to a computing device via a communication channel.

With respect to revision shoulder replacement surgery, processing circuitry 742 may then determine a recommendation for the replacement humeral implant type based on the bone density information. For example, processing circuitry 742 may determine the recommendation to be a stemless humeral implant when the bone density information indicates or represents that the density of the trabecular bone is high enough to support a stemless humeral implant. The recommendation may be based on a selection algorithm (e.g., one or more tables, equations, or machine learning algorithm such as a neural network) that is developed, perhaps by processing circuitry 742, based on historical data related to patients previously receiving a humeral implant. For example, historical data may include patient-specific image data (e.g., CT data) and the type of humeral implant (e.g., stemless or stemmed) that was previously inserted for the respective patient's prior shoulder replacement surgery or selected by the surgeon for the respective patient (e.g., via use of a thumb test to determine trabecular bone quality, or density, in the humeral head) for this revision surgery. In one example, a table may map voxel intensities, or groups of voxel intensities, to recommendations of stemmed or stemless implant types. In another example, a first table may map voxel intensities to density values, and a second table may map density values to recommendations of stemmed or stemless implant types). The system may use this mapping of image data to implant selection to inform the recommendation of implant type for a new patient based on that patient's image data. Processing circuitry 742 may then output the recommendation of the replacement humeral implant type. The recommendation may be transmitted for use in another recommendation or displayed to a user.

With respect to determining a recommendation for shoulder treatment based on bone density determined from patient-specific image data and, in some instances, soft tissue structures also determined from patient-specific image data. Processing circuitry 742 of system 740 will be described as performing this determination, but other devices or systems, such as system 742 or virtual planning system 102, may perform one or more portions of this technique. Processing circuitry 742 may determine characteristics of one or more bone structures based on patient-specific image data. Processing circuitry 742 may compute bone density measurements for at least a portion of the humeral head based on intensities of the patient-specific image data as described herein.

Processing circuitry 742 may determine one or more recommendations for shoulder treatment based on the bone density measurements. For example, processing circuitry 742 may determine whether the shoulder replacement should be a reverse or an anatomical replacement based on one or more of the bone density measurements. In addition, processing circuitry 742 may determine whether the replacement humeral implant type used in the shoulder replacement should be a stemless or stemmed humeral implant type. In some examples, processing circuitry 742 may determine the location for at least one of the replacement humeral implant or the replacement glenoid implant based on the bone density measurements. Processing circuitry 742 may then output the determined one or more recommendations for the treatment of the patient's shoulder. In this manner, processing circuitry 742 may use any of the characteristics, metrics, or other information derived from patient-specific image data and other patient information in order to provide recommendations related to shoulder treatment.

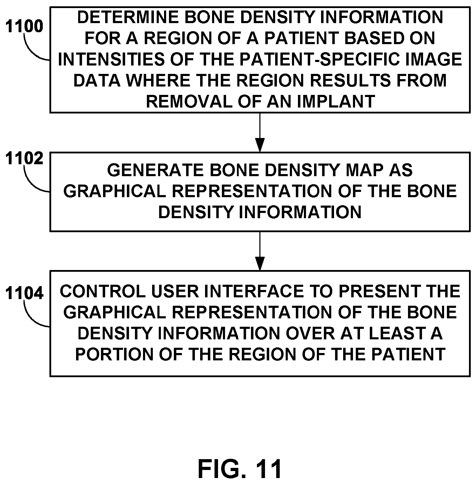

FIG. 11 is a flowchart illustrating an example procedure for displaying bone density information. Processing circuitry 742 of system 740 will be described as performing the example of FIG. 11, but other devices or systems, such as virtual planning system 102, may perform one or more portions of this technique. Furthermore, some portions of this technique may be performed by a combination of two or more devices and/or systems via a distributed system. The process of FIG. 11 is described with respect to three-dimensional data sets, but several two-dimension slices of data may be analyzed in a similar manner in other examples.

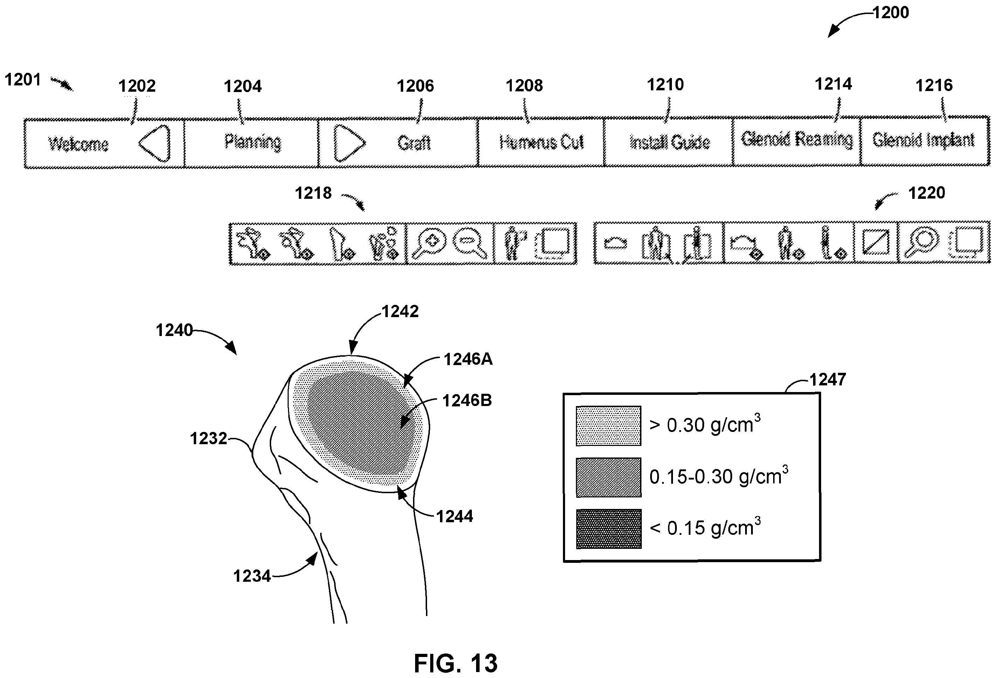

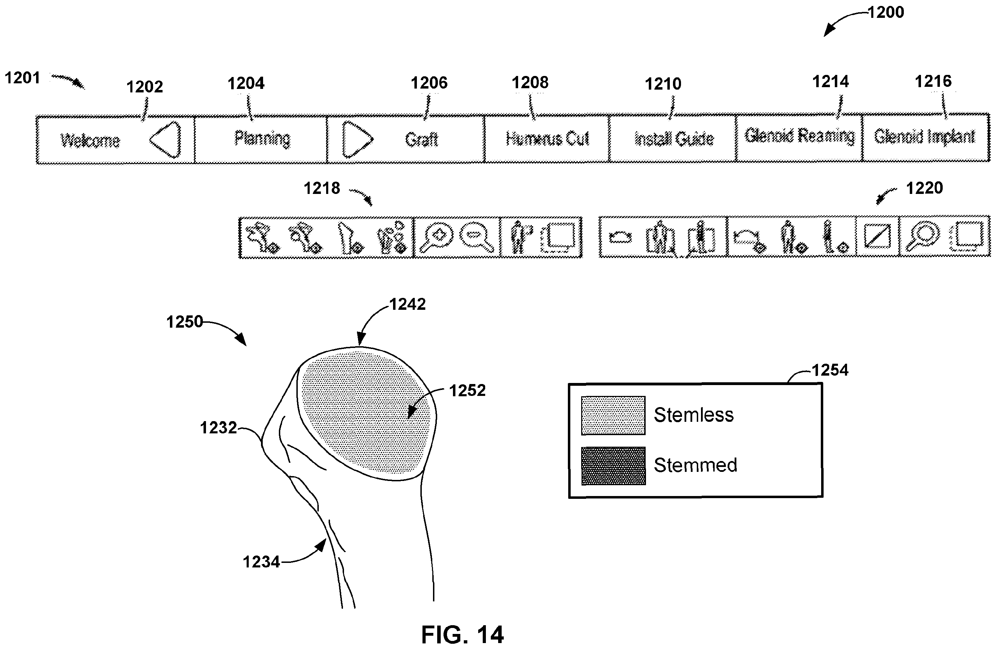

As shown in the example of FIG. 11, processing circuitry 742 may determine bone density information for at least a portion of the humeral head based on intensities of the patient-specific image data (1100), such as the process described in FIG. 9. Processing circuitry 742 may then generate a bone density map as a graphical representation of the bone density information (1102). These graphical representations may be similar to the graphical representations of the bone density metrics described in FIGS. 13 and 14. Then, processing circuitry 742 may control the user interface to present the graphical representation of the bone density metric over at least a portion of the humeral head (1104).

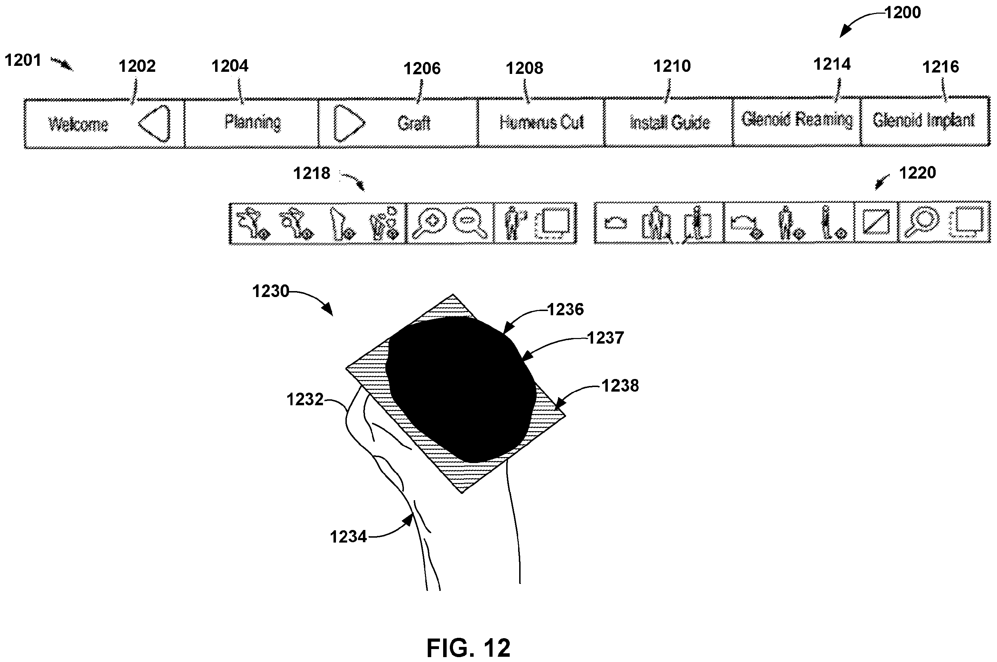

FIG. 12 is a conceptual diagram of an example user interface 1200 that includes a humerus 1232 and cut plane 1238. As shown in the example of FIG. 12, user interface 1200 includes navigation bar 1201 and toolbars 1218 and 1220. Navigation bar 1201 may include selectable buttons that, when selected by the user, cause user interface 1200 to change to a different functionality or view of information related to a shoulder treatment, such as planning a shoulder replacement.

Navigation bar 1201 may include a welcome button 1202 that takes the user to a welcome screen showing information related to the patient or possible actions related to types of treatment. Planning button 1204 may change the view of user interface 120 to virtual planning of the shoulder surgery, which may include representations of bones and/or soft tissue structures, such as view 1230 that includes humerus 1232. Graft button 1206 may show a view of potential bone grafts related to surgery, and humerus cut button 1208 may show a representation of humeral head 1232 cut to expose the trabecular bone within. Install guide button 1210 may show possible, or recommended, humeral implants. Glenoid reaming button 1214 may show a view of example reaming to be performed on the glenoid, and glenoid implant button 1216 may show examples of possible, or recommended, glenoid implants that may be implanted for the patient. Toolbar 1218 may include selectable buttons that, when selected, cause user interface 1200 to change the view, rotation, or size of view 1230. Toolbar 1220 may include selectable buttons that, when selected, cause user interface 1200 to change between anatomical planes of the anatomy shown in view 1230, such as ventral or lateral views of the anatomy.

View 1230 includes a perspective view of humerus 1232 which shows shaft 1234 and humeral head 1236 with implant 1237. Cut plane 1238 is shown to indicate how humeral head 1236 can be cut, simulating implant 1237 removal prior to implanting the humeral implant to replace implant 1237 with bone density information. Orthopedic surgical system 100 may determine an initial location of cut plane 1238 based upon implant 1237. Cut plane 1238 may be an example representation that is output onto patient-specific image data and configured to identify locations for applying a surgical instrument. User interface 1200 may enable a user to move cut plane 1238 as desired during the planning process, although user interface 1200 may initially show a recommended position for cut plane 1238. Once the user is satisfied with the position of cut plane 1238, user interface 1200 can remove the top potion of humeral head 1236 to expose a representation of trabecular bone at which a humeral implant may be implanted, as shown in FIGS. 13 and 14.

In one example, orthopedic surgical system 100 may receive patient image data (e.g., computed tomography (CT) that includes X-ray images, magnetic resonance imaging (MRI) images, or other imaging modality) and construct a three-dimensional (3D) image data set. From this image data set, orthopedic surgical system 100 can identify locations of bones associated with soft tissue structures and approximate locations of the soft tissue structures themselves. For instance, if the patient may need a shoulder replacement surgery, the system may identify parts of the scapula and humerus and muscles of the rotator cuff.

For each of the soft tissue structures (e.g., for each muscle of the rotator cuff), the system may determine a representation of the soft tissue structure from the image data. The system may place an initial shape within the estimated location of the soft tissue structure and then fit this initial shape to the image data to determine the representation of the actual soft tissue structure. This estimated location may be based on one or more markers or landmarks (e.g., muscle insertion points or muscle origins) on associated bones or other bone structures or portions of bone structures. The initial shape may be a statistical mean shape (SMS) derived from a population of subjects or any geometric shape.

From the initial shape, orthopedic surgical system 100 may use vectors normal to the surface of the initial shape to identify voxels outside or inside of the initial shape that exceed an intensity threshold representative of a boundary of the soft tissue structure within the image data. In some examples, the boundary of the soft tissue structure may be estimated from a separation zone identified between adjacent soft tissue structures. From the respective locations on the initial shape for each vector, the system may move the surface of the initial shape towards respective voxels of the identified voxels. This movement of the surface of the initial shape may occur over several iterations until the initial shape has been modified to approximate contours of the identified voxels. In other examples, orthopedic surgical system 100 may use correspondences from the initial shape to associated bones and/or minimization or maximization algorithms (e.g., a cost function) to fit and scale the initial shape to the patient-specific image data. Orthopedic surgical system 100 then removes the soft-tissue structures from the patient-specific image data. The final modified shape may then be used as the representation of the bone structure, such as the scapula and humerus, without soft tissue structure, such as a muscle of the rotator cuff of the patient as depicted in FIG. 12.

Orthopedic surgical system 100 may determine one or more qualities/characteristics of one or more bone structures from the determined representation. Virtual planning system 102 of orthopedic surgical system 100 may then be configured to use these bone qualities and/or characteristics derived from the patient image data to select or suggest certain types of medical interventions or revisions, types of surgical treatments, or even types, dimensions, and/or placement of one or more implants. In this manner, the orthopedic surgical system 100 may use the bone density information derived from the patient image data to determine or assist in the determination of surgical planning for a specific patient. For example, the system may select between an anatomical shoulder replacement surgery or a reverse shoulder replacement surgery, and then output the selection to a user such as a surgeon, e.g., by presentation on a display, based on the bone density and other qualities/characteristics derived from the patient image data. These recommendations for shoulder replacement described herein may be applied to revision surgery in which the patient has already had a shoulder replacement. Typically, a shoulder surgery may be used to restore shoulder function and/or reduce pain for a patient.

In some examples, orthopedic surgical system 100 may determine bone density information of a humeral head of a humerus based on patient-specific image data (e.g., 2D or 3D image data). For example, the system may characterize assign bone density values for voxels or groups of voxels of the trabecular bone within at least a portion of the humeral head. In other examples, the system may determine an overall bone density metric or score indicative of the entire volume of trabecular bone in at least a portion of the humeral head. The system may control a display device to display a user interface that include a representation of the bone density, such as a graphical indication of the bone density. In some examples, the system may generate a recommendation of a type of humeral implant (e.g., stemmed or stemless) based on the determined bone density. In some examples, the recommendation of the type of humeral implant may be based on historical surgical data for humeral implants in which the system has correlated the type of humeral implant used for a patient with bone density values identified in the patient-specific image data for that same patient.

FIG. 13 is a conceptual diagram of an example user interface 1200 that includes a humeral head 1242 and a representation of bone density measurement 1244. As shown in the example of FIG. 13, user interface 1200 may include view 1240 in which humeral head 1242 is shown after removal of the top of the humeral head along the cut plane 1238 of FIG. 13. Humeral head 1242 is a representation of the patient's humerus and may be derived from the patient-specific image data. Bone density measurement 1244 may be a graphical representation of the bone density metric generated for the trabecular bone of humerus 1232.

Bone density measurement 1244 may include different colors that represent voxels of intensity that fall within respective ranges 1246A and 1246B of intensities for each color. In this manner, bone density measurement 1244 may include bone density values for different groups of voxels of the trabecular bone within humeral head 1242. For example, range 1246A is representation of bone density greater than 0.30 g/cm³, and range 1246B is a representation of bone density between 0.15 g/cm³ and 0.30 g/cm³. Bone density key 1247 indicates the different colors for possible ranges of bone densities as determined from the patient-specific image data. The three ranges shown in bone density key 1247 are merely examples, and a different number of ranges or ranges having different lower and upper bounds may be used in other examples.

In other examples, view 1240 may present bone density measurement 1244 that is an image representing ranges of voxel intensities from the patient-specific image data or a representation of intensities from individual or groups of voxels. As one example, bone density measurement 1244 may simply include the voxel intensities from the patient-specific image data that correspond to the same cut plane 1238. In other words, view 1240 may include a picture of the CT data for the 2D plane corresponding to the cut plane 1238 overlaid on the exposed representation of humerus 1232. As another example, view 1240 may include heat map with different colors or patterns, for example, that correspond to different ranges of Hounsfield Units (for the example of CT data). In this manner, although the bone density metric, such as bone density measurement 1244, may be related or representative of bone density, the actual bone density metric may not actually reflect a measure of density of bone in that area.

FIG. 14 is a conceptual diagram of an example user interface 1200 that includes a humeral head 1242 and a representation of bone density measurement 1252 associated with a type of humeral implant recommendation. As shown in the example of FIG. 14, user interface 1200 may include view 1250 in which humeral head 1242 is shown after removal of the top of the humeral head along the cut plane 1238 of FIG. 12, similar to FIG. 12. Humeral head 1242 is a representation of the patient's humerus and may be derived from the patient-specific image data. Bone density measurement 1252 may be a graphical representation of the bone density measurement generated for the trabecular bone of humerus 1232.

Bone density measurement 1252 indicates the type of humeral implant that could be implanted in the trabecular bone based on the bone density determined for humerus 1232. In this manner, bone density measurement 1252 includes the determined bone density from patient-specific patient data as part of a category associated with the type of humeral implant supported by the density of the bone in humerus 1232. Metric key 1254 indicates the colors of bone density measurement 1252 that correspond to respective types of humeral implant. For example, the lighter color indicates that a stemless humeral implant can be implanted, and the darker color indicates that a stemmed humeral implant can be implanted in humerus 1232. As shown in the example of FIG. 14, bone density measurement 1252 indicates that the density of the trabecular bone is sufficient to support implantation of a stemless humeral implant. In some examples, bone density measurement 1252 may differentiate between different types of humeral implants by different colors, patterns, shapes, or other graphical representations. In one example, bone density measurement 1252 may even be a graphical representation of the type of humeral implant itself, such as an image representing the length of the stem, or stemless type, for the humeral implant.

Orthopedic surgical system 100 described herein may compute bone density measurement 1252 for at least a portion of a humeral head of a patient based on the patient-specific image data for that patient. For example, a bone density metric may be a single indication of overall density of the humeral head or a portion of the humeral head. As another example, the bone density measurement may include bone density values for respective portions of a humeral head of the patient. The bone density measurement 1252 may not actually indicate the density of bone, but may be a measurement representative of bone density (e.g., voxel intensity from image data, standard deviations of voxel intensity from image data, compressibility, etc.) Orthopedic surgical system 100 may control example user interface 1200 to present a graphical representation of the bone density measurement 1252 and/or generate a recommendation on the implant type for the humeral head based on the bone density measurement 1252. For example, a bone density measurement indicative of sufficient trabecular bone density in the humeral head may result in orthopedic surgical system 100 recommending a stemless humeral implant (the lighter color) as opposed to a stemmed humeral implant (the darker color).

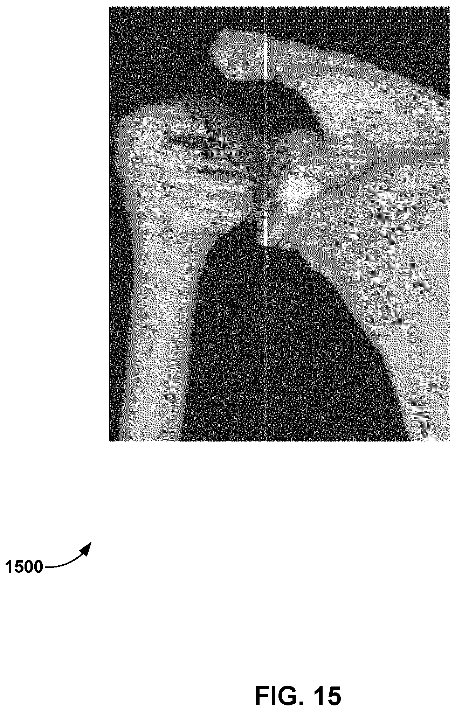

FIG. 15 is an illustration of bone density map 1500 for use by an MR system when providing revision guidance for a shoulder replacement. Bone density map 1500 indicates bone density information for areas of bone structure; in particular, bone density map 1500 provides bone density information for bone structures that form or surround a shoulder joint, such as a clavicle (collarbone), a scapula (shoulder blade), and a humerus (upper arm bone). Bone density map 1500 depicts, using black and white, bone quality/characteristics in gray scale for these bone structures.

Although depicted in gray scale between black and white, some examples of bone density map 1500 include additional colors. The purpose of these colors may be to indicate different bone qualities/characteristics. In some examples, pixel intensity values may correspond to different classes or values of bone qualities/characteristics. One color may represent "good" bone while another color may represent "bad" bone and intermediate colors represent bone qualities/characteristics in between "good" and "bad" bones.

The orthopedic surgical system 100 described herein may compute a bone density measurement for at least a portion of a humeral head of a patient based on the patient-specific image data for that patient. For example, a bone density metric may be a single indication of overall density of the humeral head or a portion of the humeral head. As another example, the bone density measurement may include bone density values for respective portions of a humeral head of the patient. The bone density metric may not actually indicate the density of bone, but may be a metric representative of bone density (e.g., voxel intensity from image data, standard deviations of voxel intensity from image data, compressibility, etc.)

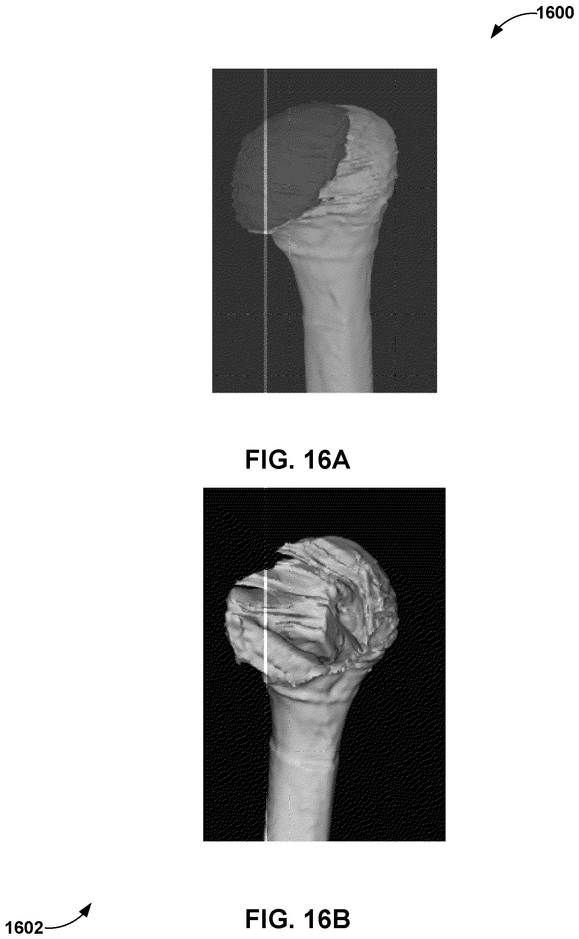

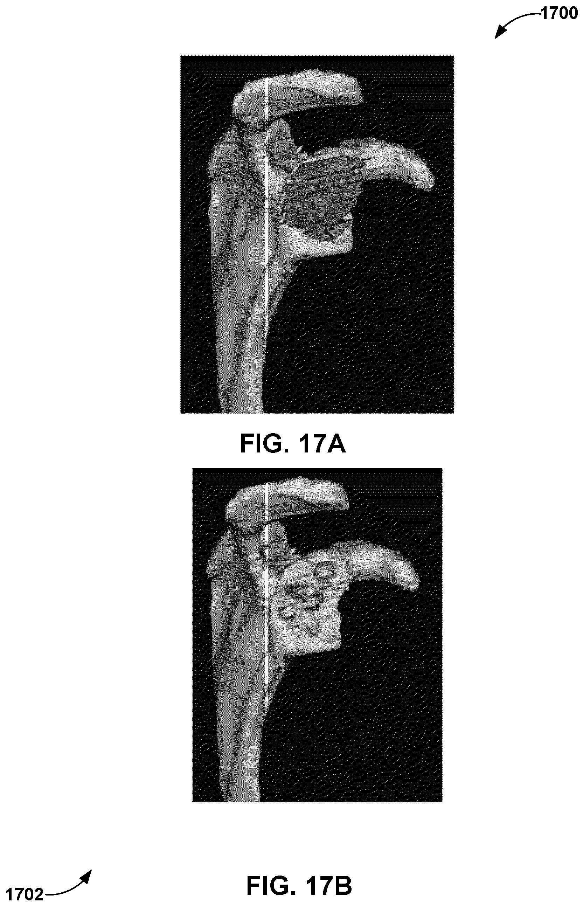

Orthopedic surgical system 100 computes, as an example, Hounsfield units which are values measuring bone density. Hounsfield units that exceed a threshold may be classified as "good" or healthy bone while Hounsfield units below another threshold or the same threshold may be classified as impaired or "bad" bone. Based upon Hounsfield units, orthopedic surgical system 100 may generate a representation indicating bone qualities/characteristics in a bone structure. As one example, in CT image data, pixels are associated with a relative radiodensity value corresponding to a mean attenuation, as measured in Hounsfield units (HUs) using the Hounsfield scale. These HU values are an example of raw image data. A visualization device converts the HU values into gray scale for display, such as in FIG. 15 as well as in FIGS. 16A-B and FIGS. 17A-B.

FIG. 16A is an illustration of bone density map 1600 for a humerus with an implant and FIG. 16B is an illustration of bone density map 1602 for a humerus without an implant. The illustration of FIG. 16A is of the humerus before a first set of patient-specific image data in that illustration undergoes an imaging process to remove an implant and replace that implant's representation with image data indicating bone density information, generating a second set of patient-specific image data. The illustration of FIG. 16B is of the humerus after the illustration of FIG. 16A undergoes an imaging process to remove the implant and replace that implant's representation with image data indicating bone density information. The imaging process exposes bone structure areas below a cut plane in the humerus and inserts bone density measurements indicating different bone qualities/characteristics of those areas of bone.

FIG. 17A is an illustration of bone density map 1700 for a scapula with an implant and FIG. 17B is an illustration of bone density map 1702 for a scapula without an implant. Similar to FIG. 16A, the illustration of FIG. 17A is of the scapula before a first set of patient-specific image data in that illustration undergoes an imaging process to remove an implant and replace that implant's representation with image data indicating bone density information, generating a second set of patient-specific image data. The illustration of FIG. 17B is of the scapula after the illustration of FIG. 17A undergoes an imaging process to remove the implant and replace that implant's representation with image data indicating bone density information. The imaging process exposes bone structure areas below a determined cut plane in the scapula and inserts bone density measurements indicating different bone qualities/characteristics of those areas of bone.

Any one or more of these bone qualities/characteristics may be used in treatment planning for a patient. The techniques described in this disclosure may also be used in the context of other types of treatment. For example, treatment for other joint disorders may be analyzes, such as a total ankle arthroplasty or other joints. While the techniques been disclosed with respect to a limited number of examples, those skilled in the art, having the benefit of this disclosure, will appreciate numerous modifications and variations there from. For instance, it is contemplated that any reasonable combination of the described examples may be performed.

It is to be recognized that depending on the example, certain acts or events of any of the techniques described herein can be performed in a different sequence, may be added, merged, or left out altogether (e.g., not all described acts or events are necessary for the practice of the techniques). Moreover, in certain examples, acts or events may be performed concurrently, e.g., through multi-threaded processing, interrupt processing, or multiple processors, rather than sequentially.

In one or more examples, the functions described may be implemented in hardware, software, firmware, or any combination thereof. If implemented in software, the functions may be stored on or transmitted over as one or more instructions or code on a computer-readable medium and executed by a hardware-based processing unit. Computer-readable media may include computer-readable storage media, which corresponds to a tangible medium such as data storage media, or communication media including any medium that facilitates transfer of a computer program from one place to another, e.g., according to a communication protocol. In this manner, computer-readable media generally may correspond to (1) tangible computer-readable storage media which is non-transitory or (2) a communication medium such as a signal or carrier wave. Data storage media may be any available media that can be accessed by one or more computers or one or more processors to retrieve instructions, code and/or data structures for implementation of the techniques described in this disclosure. A computer program product may include a computer-readable medium.

By way of example, and not limitation, such computer-readable storage media can comprise RAM, ROM, EEPROM, CD-ROM or other optical disk storage, magnetic disk storage, or other magnetic storage devices, flash memory, or any other medium that can be used to store desired program code in the form of instructions or data structures and that can be accessed by a computer. Also, any connection is properly termed a computer-readable medium. For example, if instructions are transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio, and microwave are included in the definition of medium. It should be understood, however, that computer-readable storage media and data storage media do not include connections, carrier waves, signals, or other transitory media, but are instead directed to non-transitory, tangible storage media. Disk and disc, as used herein, includes compact disc (CD), laser disc, optical disc, digital versatile disc (DVD), floppy disk and Blu-ray disc, where disks usually reproduce data magnetically, while discs reproduce data optically with lasers. Combinations of the above should also be included within the scope of computer-readable media.

Operations described in this disclosure may be performed by one or more processors, which may be implemented as fixed-function processing circuits, programmable circuits, or combinations thereof, such as one or more digital signal processors (DSPs), general purpose microprocessors, application specific integrated circuits (ASICs), field programmable gate arrays (FPGAs), or other equivalent integrated or discrete logic circuitry. Fixed-function circuits refer to circuits that provide particular functionality and are preset on the operations that can be performed. Programmable circuits refer to circuits that can programmed to perform various tasks and provide flexible functionality in the operations that can be performed. For instance, programmable circuits may execute instructions specified by software or firmware that cause the programmable circuits to operate in the manner defined by instructions of the software or firmware. Fixed-function circuits may execute software instructions (e.g., to receive parameters or output parameters), but the types of operations that the fixed-function circuits perform are generally immutable. Accordingly, the terms "processor" and "processing circuity," as used herein may refer to any of the foregoing structures or any other structure suitable for implementation of the techniques described herein.

## Claims

1. A system (740) for modeling bone density information, the system comprising:
a memory (754) configured to store a first set of patient-specific image data, the first set of patient-specific image data including image data for an implant implanted in a region of a patient; and
processing circuitry (742) configured to:
segment the implant from the region in the first set of patient-specific image data;
generate a second set of patient-specific image data based on removal of segments of the implant from the first set of patient-specific image data;
generate the bone density information for the region based on the second set of patient-specific image data;
identify areas in the second set of patient-specific image data based upon the bone density information; and
output the identified areas to at least one display device (748);
wherein the processing circuitry is further configured to:
output, to a subsystem of an orthopedic surgical system, a recommendation as to which of at least one of an implant type, a treatment type, or a surgical instrument to use for a revision surgery, the recommendation being based on the bone density information.

2. The system of claim 1, wherein the processing circuitry is further configured to, one of:
output, to a visualization device (213) of a mixed reality (MR) system (212), the bone density information corresponding to the identified areas; or
output, to the visualization device of a mixed reality (MR)system (212), the bone density information overlaying the identified areas.

3. The system of claim 1, wherein the processing circuitry further configured to:
output, to the at least one display device (748), locations for applying a surgical instrument, wherein the locations are on at least a portion of the identified areas.

4. The system of claim 1, wherein the processing circuitry is further configured to, one of:
output, to the at least one display device, a surgical plan comprising locations into which a surgical instrument is applied or an implant is implanted; or
output, to the at least one display device, a revision plan comprising locations into which at least one of an implant is removed or a replacement implant is implanted.

5. The system of claim 1, wherein the processing circuitry further is configured to:
identify, from the second set of patient-specific image data, the areas in the region corresponding to a bone density measurement that is greater than a threshold for sufficient bone density.

6. The system of claims 1, wherein the processing circuitry is further configured to:
determine, from the second set of patient-specific image data, bone density measurements for portions of the region; and
based upon the bone density measurements, classify at least one of the portions as a bone quality or characteristic.

7. The system of claim 1, wherein the processing circuitry further configured to: in the second set of patient-specific image data, output, to the at least one display device, at least one of a representation of a first bone density classification for the identified areas or a representation of a second bone density classification of the identified areas, wherein the first bone density classification comprises sufficient bone density and the second density classification comprises insufficient bone density.

8. The system of claim 7, wherein the representation of the first bone density classification comprises a first color and the representation of the second bone density classification comprises a second color.

9. The system of claims 1, wherein the processing circuitry is further configured to:
output, to the at least one display device, a bone density map as a representation of the bone density information of the identified areas.

10. The system of claim 1, wherein the processing circuitry further is configured to:
output, to a visualization device of a mixed reality (MR) system (212), a bone density map comprising representations of bone density measurements for the identified areas of the region of the patient.

11. A method for modeling bone density information, the method comprising:
storing, in a memory (754), a first set of patient-specific image data, the first set of patient-specific image data including image data for an implant implanted in a region of a patient;
segmenting the implant from the region in the first set of patient-specific image data;
generating a second set of patient-specific image data based on removal of segments of the implant from the first set of patient-specific image data;
generating the bone density information for the region based on the second set of patient-specific image data;
identifying areas in the second set of patient-specific image data based upon the bone density information; and
outputting the identified areas to at least one display device (748),
wherein outputting the identified areas further comprises:
outputting, to a subsystem of an orthopedic surgical system, a recommendation as to which of at least one of an implant type, a treatment type, or a surgical instrument to use for a revision surgery, the recommendation being based on the bone density information.

12. The method of claim 11, wherein outputting the identified areas further comprises one of:
outputting, to the at least one display device, a surgical plan comprising locations into which a surgical instrument is applied or an implant is implanted; or
outputting, to the at least one display device, a revision plan comprising locations into which at least one of an implant is removed or a replacement implant is implanted.

13. The method of claim 11, wherein outputting the identified areas further comprises one of:
outputting, to the at least one display device, a bone density map as a representation of the bone density information of the identified areas; or
outputting, to the at least one display device, locations for applying a surgical instrument, wherein the locations are on at least a portion of the identified areas.

14. A computer readable storage medium comprising instructions that, when executed by processing circuitry, causes the processing circuitry to perform the method of claim 11.

## Patentansprüche

1. System (740) zum Modellieren von Knochendichteinformationen, wobei das System Folgendes umfasst:
einen Speicher (754), der konfiguriert ist, um einen ersten Satz patientenspezifischer Bilddaten zu speichern, wobei der erste Satz patientenspezifischer Bilddaten Bilddaten für ein Implantat aufweist, das in einer Region eines Patienten implantiert ist; und
eine Verarbeitungsschaltlogik (742), die konfiguriert ist zum:
Segmentieren des Implantats aus der Region in dem ersten Satz patientenspezifischer Bilddaten;
Erzeugen eines zweiten Satzes patientenspezifischer Bilddaten basierend auf dem Entfernen von Segmenten des Implantats aus dem ersten Satz patientenspezifischer Bilddaten;
Erzeugen der Knochendichteinformationen für die Region basierend auf dem zweiten Satz patientenspezifischer Bilddaten;
Identifizieren von Bereichen in dem zweiten Satz patientenspezifischer Bilddaten basierend auf den Knochendichteinformationen; und
Ausgeben der identifizierten Bereiche an mindestens eine Anzeigevorrichtung (748);
wobei die Verarbeitungsschaltlogik ferner konfiguriert ist zum:
Ausgeben einer Empfehlung an ein Subsystem eines orthopädischen chirurgischen Systems darüber, welches von mindestens einem von einem Implantattyp, einem Behandlungstyp oder einem chirurgischen Instrument für eine Revisionsoperation zu verwenden ist, wobei die Empfehlung auf den Knochendichteinformationen basiert.

2. System nach Anspruch 1, wobei die Verarbeitungsschaltlogik ferner konfiguriert ist zu einem von:
Ausgeben der den identifizierten Bereichen entsprechenden Knochendichteinformationen an eine Visualisierungsvorrichtung (213) eines Mixed-Reality-(MR-)Systems (212); oder
Ausgeben der Knochendichteinformationen, die die identifizierten Bereiche überlagern, an die Visualisierungsvorrichtung eines Mixed-Reality-(MR-)Systems (212).

3. System nach Anspruch 1, wobei die Verarbeitungsschaltlogik ferner konfiguriert ist zum: Ausgeben von Stellen zum Ansetzen eines chirurgischen Instruments an die mindestens eine Anzeigevorrichtung (748), wobei sich die Stellen auf mindestens einem Abschnitt der identifizierten Bereiche befinden.

4. System nach Anspruch 1, wobei die Verarbeitungsschaltlogik ferner konfiguriert ist zu einem von:
Ausgeben eines chirurgischen Plans, der Stellen umfasst, in die ein chirurgisches Instrument eingebracht wird oder ein Implantat implantiert wird, an die mindestens eine Anzeigevorrichtung; oder
Ausgeben eines Revisionsplans, der Stellen umfasst, an denen mindestens ein Implantat entfernt wird oder ein Ersatzimplantat implantiert wird, an die mindestens eine Anzeigevorrichtung.

5. System nach Anspruch 1, wobei die Verarbeitungsschaltlogik ferner konfiguriert ist zum: Identifizieren, aus dem zweiten Satz von patientenspezifischen Bilddaten, der Bereiche in der Region, die einer Knochendichtemessung entsprechen, die größer als ein Schwellenwert für eine ausreichende Knochendichte ist.

6. System nach Anspruch 1, wobei die Verarbeitungsschaltlogik ferner konfiguriert ist zum:
Bestimmen von Knochendichtemesswerten für Abschnitte der Region aus dem zweiten Satz patientenspezifischer Bilddaten; und
Klassifizieren mindestens eines der Abschnitte basierend auf den Knochendichtemessungen als eine Knochenqualität oder -eigenschaft.

7. System nach Anspruch 1, wobei die Verarbeitungsschaltlogik ferner konfiguriert ist zum: Ausgeben, in dem zweiten Satz von patientenspezifischen Bilddaten, mindestens einer von einer Darstellung einer ersten Knochendichteklassifizierung für die identifizierten Bereiche oder einer Darstellung einer zweiten Knochendichteklassifizierung der identifizierten Bereiche an die mindestens eine Anzeigevorrichtung, wobei die erste Knochendichteklassifizierung eine ausreichende Knochendichte umfasst und die zweite Dichteklassifizierung eine unzureichende Knochendichte umfasst.

8. System nach Anspruch 7, wobei die Darstellung der ersten Knochendichteklassifizierung eine erste Farbe umfasst und die Darstellung der zweiten Knochendichteklassifizierung eine zweite Farbe umfasst.

9. System nach Anspruch 1, wobei die Verarbeitungsschaltlogik ferner konfiguriert ist zum: Ausgeben einer Knochendichtekarte als eine Darstellung der Knochendichteinformationen der identifizierten Bereiche an die mindestens eine Anzeigevorrichtung.

10. System nach Anspruch 1, wobei die Verarbeitungsschaltlogik ferner konfiguriert ist zum: Ausgeben einer Knochendichtekarte, die Darstellungen von Knochendichtemessungen für die identifizierten Bereiche der Region des Patienten umfasst, an eine Visualisierungsvorrichtung eines Mixed-Reality-(MR-)Systems (212).

11. Verfahren zum Modellieren von Knochendichteinformationen, wobei das Verfahren umfasst:
Speichern eines ersten Satzes patientenspezifischer Bilddaten in einem Speicher (754), wobei der erste Satz patientenspezifischer Bilddaten Bilddaten für ein Implantat aufweist, das in einer Region eines Patienten implantiert ist;
Segmentieren des Implantats aus der Region in dem ersten Satz patientenspezifischer Bilddaten;
Erzeugen eines zweiten Satzes patientenspezifischer Bilddaten basierend auf dem Entfernen von Segmenten des Implantats aus dem ersten Satz patientenspezifischer Bilddaten;
Erzeugen der Knochendichteinformationen für die Region basierend auf dem zweiten Satz patientenspezifischer Bilddaten;
Identifizieren von Bereichen in dem zweiten Satz patientenspezifischer Bilddaten basierend auf den Knochendichteinformationen; und
Ausgeben der identifizierten Bereiche an mindestens eine Anzeigevorrichtung (748),
wobei das Ausgeben der identifizierten Bereiche ferner umfasst:
Ausgeben einer Empfehlung an ein Subsystem eines orthopädischen chirurgischen Systems darüber, welches von mindestens einem von einem Implantattyp, einem Behandlungstyp oder einem chirurgischen Instrument für eine Revisionsoperation zu verwenden ist, wobei die Empfehlung auf der Knochendichteinformation basiert.

12. Verfahren nach Anspruch 11, wobei das Ausgeben der identifizierten Bereiche ferner eines von Folgendem umfasst:
Ausgeben eines chirurgischen Plans, der Stellen umfasst, in die ein chirurgisches Instrument eingebracht wird oder ein Implantat implantiert wird, an die mindestens eine Anzeigevorrichtung; oder
Ausgeben eines Revisionsplans, der Stellen umfasst, an denen mindestens ein Implantat entfernt wird oder ein Ersatzimplantat implantiert wird, an die mindestens eine Anzeigevorrichtung.

13. Verfahren nach Anspruch 11, wobei das Ausgeben der identifizierten Bereiche ferner eines von Folgendem umfasst:
Ausgeben einer Knochendichtekarte als eine Darstellung der Knochendichteinformation der identifizierten Bereiche an die mindestens eine Anzeigevorrichtung; oder
Ausgeben von Stellen zum Ansetzen eines chirurgischen Instruments an die mindestens eine Anzeigevorrichtung, wobei sich die Stellen auf mindestens einem Abschnitt der identifizierten Bereiche befinden.

14. Computerlesbares Speicherungsmedium, umfassend Anweisungen, die bei Ausführung durch eine Verarbeitungsschaltlogik die Verarbeitungsschaltlogik veranlassen, das Verfahren nach Anspruch 11 durchzuführen.

## Revendications

1. Système (740) destiné à modéliser des informations de densité osseuse, le système comprenant :
une mémoire (754) configurée pour stocker un premier ensemble de données d'image spécifiques à un patient, le premier ensemble de données d'image spécifiques à un patient comportant des données d'image concernant un implant implanté dans une région du corps d'un patient ; et
un ensemble de circuits de traitement (742) configuré pour :
segmenter l'implant à partir de la région dans le premier ensemble de données d'image spécifiques à un patient ;
générer un deuxième ensemble de données d'image spécifiques à un patient en retirant les segments de l'implant du premier ensemble de données d'image spécifiques à un patient ;
générer les informations de densité osseuse concernant la région sur la base du deuxième ensemble de données d'image spécifiques à un patient ;
identifier des zones dans le deuxième ensemble de données d'image spécifiques à un patient sur la base des informations de densité osseuse ; et
délivrer les zones identifiées à au moins un dispositif d'affichage (748) ;
dans lequel l'ensemble de circuits de traitement est en outre configuré pour :
délivrer à un sous-système d'un système chirurgical orthopédique une recommandation précisant lequel utiliser parmi un type d'implant, un type de thérapie et/ou un instrument chirurgical pour une chirurgie de révision, la recommandation étant basée sur les informations de densité osseuse.

2. Système selon la revendication 1, dans lequel l'ensemble de circuits de traitement est en outre configuré pour effectuer l'une des opérations suivantes :
délivrer à un dispositif de visualisation (213) d'un système de réalité mixte (MR) (212) les informations de densité osseuse correspondant aux zones identifiées ; ou
délivrer au dispositif de visualisation d'un système de réalité mixte (MR) (212) les informations de densité osseuse superposées aux zones identifiées.

3. Système selon la revendication 1, dans lequel l'ensemble de circuits de traitement est en outre configuré pour :
délivrer à l'au moins un dispositif d'affichage (748) des emplacements d'application d'un instrument chirurgical, dans lequel les emplacements se trouvent sur au moins une partie des zones identifiées.

4. Système selon la revendication 1, dans lequel l'ensemble de circuits de traitement est en outre configuré pour effectuer l'une des opérations suivantes :
délivrer à l'au moins un dispositif d'affichage un plan chirurgical comprenant des emplacements dans lesquels un instrument chirurgical est appliqué ou dans lesquels un implant est implanté ; ou
délivrer à l'au moins un dispositif d'affichage un plan de révision comprenant des emplacements dans lesquels un implant est retiré et/ou dans lesquels un implant de remplacement est implanté.

5. Système selon la revendication 1, dans lequel l'ensemble de circuits de traitement est en outre configuré pour :
à partir du deuxième ensemble de données d'image spécifiques à un patient, identifier les zones de la région correspondant à une mesure de densité osseuse qui est supérieure à un seuil de densité osseuse suffisante.

6. Système selon la revendications 1, dans lequel l'ensemble de circuits de traitement est en outre configuré pour :
à partir du deuxième ensemble de données d'image spécifiques à un patient, déterminer des mesures de densité osseuse de parties de la région ; et
sur la base des mesures de densité osseuse, classer au moins une des parties comme une qualité ou caractéristique osseuse.

7. Système selon la revendication 1, dans lequel l'ensemble de circuits de traitement est en outre configuré pour : dans le deuxième ensemble de données d'image spécifiques à un patient, délivrer à l'au moins un dispositif d'affichage une représentation d'une première classification de densité osseuse pour les zones identifiées et/ou une représentation d'une deuxième classification de densité osseuse des zones identifiées, dans lequel la première classification de densité osseuse comprend une densité osseuse suffisante et la deuxième classification de densité comprend une densité osseuse insuffisante.

8. Système selon la revendication 7, dans lequel la représentation de la première classification de densité osseuse comprend une première couleur et la représentation de la deuxième classification de densité osseuse comprend une deuxième couleur.

9. Système selon la revendications 1, dans lequel l'ensemble de circuits de traitement est en outre configuré pour :
délivrer à l'au moins un dispositif d'affichage une carte de densité osseuse comme une représentation des informations de densité osseuse des zones identifiées.

10. Système selon la revendication 1, dans lequel l'ensemble de circuits de traitement est en outre configuré pour :
délivrer à un dispositif de visualisation d'un système de réalité mixte (MR) (212) une carte de densité osseuse comprenant des représentations de mesures de densité osseuse pour les zones identifiées de la région du patient.

11. Procédé destiné à modéliser des informations de densité osseuse, le procédé comprenant les étapes suivantes :
stocker dans une mémoire (754) un premier ensemble de données d'image spécifiques à un patient, le premier ensemble de données d'image spécifiques à un patient comportant des données d'image concernant un implant implanté dans une région du corps d'un patient ;
segmenter l'implant à partir de la région dans le premier ensemble de données d'image spécifiques à un patient ;
générer un deuxième ensemble de données d'image spécifiques à un patient en retirant les segments de l'implant du premier ensemble de données d'image spécifiques à un patient ;
générer les informations de densité osseuse concernant la région sur la base du deuxième ensemble de données d'image spécifiques à un patient ;
identifier des zones dans le deuxième ensemble de données d'image spécifiques à un patient sur la base des informations de densité osseuse ; et
délivrer les zones identifiées à au moins un dispositif d'affichage (748),
dans lequel le fait de délivrer les zones identifiées comprend en outre l'opération suivante :
délivrer à un sous-système d'un système chirurgical orthopédique une recommandation précisant lequel utiliser parmi un type d'implant, un type de thérapie et/ou un instrument chirurgical pour une chirurgie de révision, la recommandation étant basée sur les informations de densité osseuse.

12. Procédé selon la revendication 11, dans lequel le fait de délivrer les zones identifiées comprend en outre l'une des étapes suivantes :
délivrer à l'au moins un dispositif d'affichage un plan chirurgical comprenant des emplacements dans lesquels un instrument chirurgical est appliqué ou dans lesquels un implant est implanté ; ou
délivrer à l'au moins un dispositif d'affichage un plan de révision comprenant des emplacements dans lesquels un implant est retiré et/ou dans lesquels un implant de remplacement est implanté.

13. Procédé selon la revendication 11, dans lequel le fait de délivrer les zones identifiées comprend en outre l'une des opérations suivantes :
délivrer à l'au moins un dispositif d'affichage une carte de densité osseuse comme une représentation des informations de densité osseuse des zones identifiées ; ou
délivrer à l'au moins un dispositif d'affichage des emplacements d'application d'un instrument chirurgical, dans lequel les emplacements se trouvent sur au moins une partie des zones identifiées.

14. Support de stockage lisible par ordinateur comprenant des instructions qui, lorsqu'elles sont exécutées par un ensemble de circuits de traitement, amènent l'ensemble de circuits de traitement à mettre en œuvre le procédé selon la revendication 11.
